# EUROPEAN PATENT APPLICATION

(11) **EP 4 108 188 A1**
(43) Date of publication of application: **28.12.2022**
(21) Application number: 21315103.8
(22) Date of filing: 23.06.2021
(51) Int. Cl.: A61B 17/221, A61B 34/00, A61B 17/22

(54) **MEDICAL DEVICE, SYSTEM AND METHOD FOR RETRIEVING A THROMBUS FROM A VESSEL, AND METHOD FOR PRODUCING A DEVICE**

(71) Applicant: Artedrone, 75003 Paris (FR)
(72) Inventor: Pouponneau, Pierre, 75015 Paris (FR); Bruneau, Maëlle, 75011 Paris (FR)
(74) Representative: Hepp Wenger Ryffel AG

(57) **Abstract**

The invention is directed to a medical device (1) for retrieving a thrombus (2) from a vessel (3). The medical device (1) comprises an attachment element (4) arranged at a distal end (5) of the medical device (1). The attachment element (4) is adapted to attach to a proximal face (6) of the thrombus such that the thrombus (2) is retrievable by exerting a pulling force on the attachment element (4).

## Description

The present invention relates to a medical device, a system and a method for retrieving a thrombus from a vessel, and to a method of producing a device according to the preamble of the independent claims.

It is known in the prior art to use medical devices to remove thrombi from vessels.

For example, US 2017/119407 discloses aspiration devices for removal of blood clots.

US 2013/060269 discloses a stent device, which penetrates a material in order to anchor it.

US 2013/289578 discloses a catheter device for surrounding and gripping blood clots.

However, devices and methods known in the art have several drawbacks. For example, known devices are typically large, which may limit the accessible locations in a vessel system and increase the risk of unwanted interaction with tissue which may lead to injuries. Furthermore, large devices may be more difficult to operate by a surgeon. Furthermore, known devices may require complicated mechanisms for safe removal of a thrombus. In addition, treatments using known devices and methods may take a long time, leading to higher patient discomfort and higher risks associated with the treatment.

Thus, the object of the present invention is to overcome the drawbacks of the prior art, in particular to provide a device, a system and a method to provide easy, safe and versatile treatment of vessels.

This and other objects are achieved by the medical device, the system and the methods according to the characterizing portion of the independent claims of the invention.

The medical device according to the invention is adapted for retrieving a thrombus from a vessel. The device comprises an attachment element arranged at a distal end of the medical device. The attachment element is adapted to attach to a proximal face of the thrombus such that the thrombus is retrievable by exerting a pulling force on the attachment element.

The attachment element may be any element adapted to attach to a thrombus, in particular it may comprise structure for mechanical interaction such as forks, but also glues, fibers. The attachment element may further be connected to a main body, for example a magnetic part, in particular through a form-fit connection.

In one particularly preferred embodiment, the medical device comprises a magnetic part which is attached to a controlling line, and a connection element for an anchoring element that comprises or consists of the attachment element. The anchoring element may be connected to the magnetic part via a form-fit connection, a snap-fit connection, an adhesive connection, a bayonet connection, or any other connection means known in the art. The connection may be reversible or non-reversible.

In certain embodiments, the medical device comprises or consists of a catheter device and/or a microrobot.

Proximal attachment of the device at a thrombus is particularly advantageous because it enables a quick attachment without the need to penetrate or navigate device elements in our around the thrombus. In addition, because a pulling force is exerted on the proximal face of the thrombus, the thrombus may be stretched, which may lead to a smaller diameter, thus reducing the necessary pulling force, as the friction between the vessel wall and the thrombus may be reduced.

A proximal face of the thrombus may be understood as the proximal-most area of the thrombus in a direction towards the device, i.e. in a direction of intended removal of the thrombus.

The attachment element may comprise a biological attachment mechanism. In particular, the biological attachment mechanisms may comprise or consist of a thrombogenic element. The thrombogenic element may comprise thrombin, calcium chloride, and/or collagen. The thrombogenic element may be a dried coagulant layer.

The thrombogenic element may comprise or consist of fibers with thrombogenic properties.

Biological attachment mechanisms may provide a particularly simple way of attaching a thrombus as mechanisms inherent in a human or animal body may be capitalized on. For example, thrombogenic elements may form an attachment material from blood coagulation. Thus, a portion of the attachment material does not need to be delivered to a treatment site.

The biological attachment mechanism may be any mechanism which triggers a biological reaction with tissue and/or blood, for example coagulation.

The thrombogenic element may be delivered in a dried state, for example as a dried coagulant layer. This may enable particularly easy activation using a body fluid such as blood. Thus, the dried coagulant layer may become adhesive only when it is in contact with blood or another bodily fluid, or water or saline delivered to the treatment site.

The attachment element, preferably the biological attachment mechanism, may comprise electrical contacts attached or attachable to an electrical conductor. The medical device may thus, particularly preferably, comprise an electrical conductor attached or attachable to an energy source. The electrical contacts are adapted to create attachment through tissue change induced by temperature increase and/or electrical current/voltage.

The energy source may be electrical energy from a building installation, a converter connected thereto, a battery, or any other electrical energy supply known in the art.

Preferably, the attachment element is adapted to provide attachment to a thrombus via cauterization and/or electro-thrombosis.

The attachment element may comprise a chemical attachment mechanism. In particular, the chemical attachment mechanism may comprise an adhesive composition, preferably a dried adhesive composition.

Chemical attachment mechanisms such as adhesive compositions may be particularly advantageous as they may provide instant attachment, e.g. without time lapse for coagulum formation, and/or without further elements, e.g. it may not be necessary to deliver further/larger elements to the treatment site.

The adhesive composition may be activatable. In particular, the adhesive composition may be activatable by temperature, light, humidity, and/or a change in pH.

The attachment element may comprise a mechanical attachment mechanism, in particular a mechanical attachment mechanism comprising a shape-change element. The mechanical attachment mechanism may be selected from a group comprising a hook, a fork, a spring, and a spike.

Mechanical attachment mechanisms may be particularly safe as no chemically or biologically reactive elements need to be introduced into the body. Even if biological or chemical attachment mechanisms are combined with a mechanical attachment mechanism, mechanical attachment mechanisms can be advantageous because they may provide more secure and easily adjustable attachment.

A shape change element may in particular comprise or consist of a self-expansive material for increasing a force toward a thrombus, for example to enhance a mechanical attachment or an adhesive force.

The shape-change element may also comprise or consist of a shape memory material, for example a Nitinol alloy.

Preferably, the shape-change element is adapted to change from an axial/linear shape to a radial/three-dimensional shape, for example a helix or a hook. Additionally or alternately, the shape-change element may be configured to adopt an umbrella shape upon the change of its shape.

Particularly preferably, a mechanical anchor made of a shape memory alloy may be configured to have a straight shape adapted to change its shape upon attachment to a thrombus, in particular to a cork-screw or hook-shape.

Any shape known in the art suitable to provide attachment after penetration may be used, in particular screw shapes.

The medical device may further comprise a suction mechanism. Preferably, the attachment element comprises the suction mechanism.

The suction mechanism may assist in attachment of the attachment element to the thrombus. Additionally or alternatively, the suction mechanism may be used to remove fluids and/or tissue from the treatment site. For example, blood may be aspirated from the treatment site in order to remove thrombus debris to reduce risks for the patient. Additionally or alternatively, soft thrombus parts (such as red thrombi) may be aspirated in order to reach and attach to a more rigid part of the thrombus.

Additionally or alternatively, a suction mechanism may be used to activate a mechanical element, for example by utilizing a pneumatic and/or hydraulic mechanism. To this end, the medical device may further comprise a canal which is in operable connection with the suction mechanism such as to provide pneumatic or hydraulic line.

It will be understood that any combination of biological, chemical and/or mechanical attachment mechanisms described herein is possible. It will also be understood that an attachment mechanism may be biological, chemical and/or mechanical simultaneously. As such, a mention of one of a biological, chemical, or mechanical attachment shall not necessarily be understood as excluding the other two mechanisms unless stated otherwise. For example, a mechanical attachment mechanism may be assisted by adhesion and/or coagulation. However, it is of course possible to configure an attachment mechanism as being only mechanical, only biological, or only mechanical.

Preferably, the medical device comprises an activation mechanism. The attachment element may have an activated state and a deactivated state. In the deactivated state, the attachment element is adapted to not interact with a vessel wall or with the thrombus. In the activated state, the attachment element is adapted to interact with the thrombus. The activation mechanism is adapted to bring the attachment element at least from the deactivated state to the activated state.

An activation mechanism may be advantageous because inadvertent interaction between the attachment element and tissue and/or blood can be prevented during delivery, which may lead to safer treatments and less complications, and easier delivery.

Preferably, the medical device comprises a microrobot equipped with the attachment element.

The microrobot may comprise at least one of a magnetic part and a controlling line. The magnetic part may be adapted to interact with a, preferably external, magnetic field. Additionally or alternatively, the controlling line may be attachable or attached to a distal part of the microrobot.

The controlling line may be adapted to pull back a thrombus. In particular, it may be adapted, by material choice and/or appropriate dimensions, to have a minimum strength in the range of 0.5 N to 20N, preferably 8 N to 12 N.

The medical device may be formed by at least a part of a catheter device. Additionally or alternatively, a guide wire may also be used, or any other intravascular device.

The activation mechanism may comprise a mechanism adapted to release at least the attachment element from a storage area of the medical device.

For example, the attachment element may be stored in a compartment of a catheter or a microrobot.

Additionally or alternatively, the attachment element may be encapsulated.

The activation mechanism may comprise an activatable material.

The activatable material may be activatable by any activation mechanism described herein or known in the art. For example, the activatable material may be activatable by exposure to a magnetic field, magnetic gradient, ultrasound, or light (such as light-curable adhesives). Additionally or alternatively, the activatable material may be activated by temperature increase, exposure to humidity/water, change in pH, in response to a salt concentration, activated inflammation species, change in the blood flow velocity, electricity, or others

The activation mechanism may comprise a protection layer. The protection layer may be selected from a group comprising a protection coating, a protection sheath, a protection frame, and a protection cap.

In general, a protection layer may be removed to provide such as to bring the attachment element from a deactivated state to an activated state.

In general, the protection layer may be biodegradable or non-biodegradable, may be a cap that may be removed, or a layer that breaks apart, or comprises a hole and/or a predetermined breaking point.

A protection coating may be soluble in water or blood such as to automatically activate the attachment element after a certain exposure time in blood. The exposure time may be adapted to be in the range of, for example, 10 sec to 20 min, preferably 2 to 5 min.

The protection coating can comprise or consist of biodegradable polymers such as PGA and/or PLGA. In such a configuration, the coating is divided into parts. The natural mechanism for the degradation is the hydrolysis. Hydrolysis typically takes place within several minutes after direct contact with physiological solutions.

To achieve sufficiently fast degradation, PGA may have a very low molecular weight. Once hydrolysis of PGA has started, the mechanical strength of the biodegradable coating decreases and thus breaking of the protection coating is easier.

Preferably, the protection coating comprises or consists of a mixture (blend and/or co-polymer) of different polymers. Thus, mechanical properties as well as the degradation rate may be tuned by adjusting a mixing ratio.

Degradation may be accelerated under stimuli such as heat. A bioresorbable coating can be made of biodegradable polymer loaded with nanoparticles. The polymer exhibits a low degradation temperature of 40-60°C. The nanoparticles can be made of gold, Fe₂O₃, Fe₃O₄ and/or silver. The protective coating is degraded by heat generated by the nanoparticles under a stimulus such as light and/or a magnetic field. The coating may then break into small biodegradable parts which are flushed away by the blood flow.

In another embodiment, the protection coating comprises a membrane made of magnesium powder acting as a linker embedded in a bioresorbable polymer. The degradation of the powder is accelerated by applying a current provided by the controlling line. The degradation of the particles can contribute to the breaking of the coating.

Additionally or alternatively, the biodegradable coating can be made of magnesium or a composite which is degraded by biocorrosion. The biocorrosion process may be accelerated by applying a current.

The coating can be made of a biodegradable part and a non-biodegradable part. The non-biodegradable part remains attached to the device, in particular the attachment element. The biodegradable part may be removed from the device during the removal/degradation of the protective coating.

The coating may be made of non-biodegradable polymers such as silicon, polyurethane with polycarbonate backbone or PTFE, for example. The non-biodegradable polymer may be broken during the opening. Preferably, the non-biodegradable coating is broken by tearing in order that the coating remains attached to the device and/or the attachment element. Preferably, for a capsule, polymers such as PVDF, HDPE, PMMA, LCP, PA46, PI can be used.

A protection frame may prevent direct contact between the attachment element and a surrounding tissue until removal of the protection frame.

The medical device may further comprise an extension member. The extension member may preferably comprise or consist of a spring. The extension member may be extendable in an axial direction and may be adapted for the generation of an axial force on the thrombus.

The extension member may be arranged at a proximal or distal end of the medical device. The extension member preferably is part of the attachment element.

In particular, the axial force provided by the extension member may additionally or alternatively be adapted to be used to open and/or remove the protection layer.

The medical device may further comprise a sensor.

Preferably, the sensor is adapted to determine incorrect or insufficient attachment of the attachment element to the thrombus.

The sensor is preferably selected from the group comprising a force sensor, a temperature sensor, a pH sensor, an attachment sensor, a flow sensor, a pressure sensor, and a contact surface sensor.

For example, the sensor may comprise force sensor adapted to measure a pulling force acting on the thrombus and/or a sensor adapted to measure the contact surface between the attachment element and the thrombus.

In particular, a pressure sensor may be used to monitor whether or not a the attachment element is in contact with the thrombus and/or evaluate the nature of the thrombus.

Additionally or alternatively, the sensor may be adapted to monitor whether the attachment element is sufficiently attached to the thrombus during retrieval.

Preferably, the attachment element comprises a substantially flat surface which is adapted for attachment to the thrombus.

Substantially flat may be understood as having a limited, maximum curvature (i.e. a minimum radius of curvature). In particular, the substantially flat surface may exhibit a minimum radius of curvature of 3 cm, preferably 5 cm, particularly preferably 50 cm.

Additionally or alternatively, the attachment element may have a maximum extension in an axial direction of the medical device of 5 to 40 mm, preferably 15 to 25 mm, particularly preferably 20 mm. For example, the attachment element may be the distal-most portion of the medical device in its intended use. Alternatively, the maximum extension may be between 1 and 5 mm, preferably 2 mm.

Preferably, a size of the attachment element in its activated position when attached to the thrombus is smaller than 3 mm, particularly preferably smaller than 1 mm, even more preferably 0.8 mm, in a direction perpendicular to a longitudinal axis of the medical device. Preferably, this size is the maximum size of the attachment element perpendicular to a longitudinal axis of the medical device.

Thus, the attachment element may have a size which allows for navigation even in small vessels and thus provides more versatile treatment options.

Preferably, the size of the attachment element is between 0.2 and 5 mm, particularly preferably between 0.3 and 1 mm.

Preferably, the attachment element is smaller, in a direction perpendicular to the longitudinal axis of the medical device, than a maximum size and/or a size of any other portion of the medical device, when attached to the thrombus and/or in the activated state.

Thus, it can be ensured that any location that is accessed by the medical device can be treated using the attachment element as the attachment element will fit into the vasculature at the treatment location.

Particularly preferably, the maximum size of the medical device is a size of the magnetic part in a direction perpendicular to the longitudinal axis of the medical device.

Preferably, the attachment element is configured such that a maximum size of the attachment element in a direction perpendicular to a longitudinal axis of the medical device differs by less than 10%, preferably 5%, between the activated and the deactivated state. Particularly preferably, the maximum size of the attachment element in a direction perpendicular to a longitudinal axis of the medical device remains constant.

If the maximum size of the attachment element differs between the deactivated and the activated state, it is particularly preferred that said maximum size is larger in the activated state compared to the activated state.

The difference in said maximum size may be in an axial (i.e. along the longitudinal axis of the medical device) or radial (i.e. perpendicular to the longitudinal axis of the medical device) direction, or both.

Preferably, the medical device comprises a, preferably chemical, propulsion member generating an axial propulsion force.

The medical device, in particular the magnetic part with the attachment element, may typically be moved forward mainly by a flow force exerted by blood. When a thrombus is present, the flow can be modified or reduced. To ensure that the medical device can reach the treatment site and the proximal surface of the thrombus, an additional propulsion system may be advantageous. It will be understood that such an additional propulsion system is particularly advantageous, but not necessary to the invention, as a magnetic force exerted by a magnetic field on the magnetic part may be sufficient.

The additional propulsion system may be mechanical. For example, it may comprise a spring which can be compressed and embedded into the controlling line. The spring may be arranged between the controlling line and the magnetic part. The spring can be released by removing or degrading a physical locking mechanism such as a barrier. The spring can be made of a shape memory alloy, for example nitinol. Under the influence of a stimulus, such as heat or electricity, the spring may be activated. To activate the spring by heat, it can be coated with a material which can be heated. An example of such configuration can be a spring coated with gold nanoparticles which will be heated when exposed to, for example, infrared light. Another configuration can be a spring coated with superparamagnetic nanoparticles. These particles can be heated in an alternating magnetic field.

The additional propulsion system may alternatively be chemical. The propulsion member may comprise a reactive species. For example, magnesium may be used which, when in contact with blood, can react with H₂O by forming a gas which is used to propel the device. Typically, the additional propulsion is only required and thus activated near the treatment site, i.e. the thrombus. Hence, the reactive specie may be embedded into a tank. As an example, a tank can be made of a tubing setup around the magnetic part and attached via a shell. The tank may be closed with a plug. The plug can be mechanically removed or degraded under a stimulus. The plug can be attached to a line setup connected with the controlling line. A force acting on this line, induced for example by a controlling line driver and/or via fluid flow in a canal (e.g. pneumatic or hydraulic pressure), may remove the plug and thus the content of the tank can come in contact with blood. Additionally or alternatively, the plug may be degraded under a stimulus such as electricity (e.g. biocorrosion due to a current) or exposure to light as described herein. In such a configuration the plug may be made of metal such as tungsten and/or titanium or polymer such as silicone, PEEK, or any combination thereof. In such a configuration the outer diameter of the plug may particularly preferably be about 240 µm. The inner diameter of the tank may be 100 µm. The outer diameter of the tank may be 200 µm. The tank may be made of a polymer such as PDMS.

Preferably, the medical device comprises at least one drag member. The drag member may be activatable. The drag member is adapted for increasing forces acting on the medical device.

The drag member may, in particular, be magnetic and/or hydrodynamic, i.e. adapted to increase magnetic and/or hydrodynamic forces acting on the medical device.

For example, the drag member may comprise an umbrella-like element arranged on the magnetic part or on the controlling line. Opening of the umbrella-like element may increase the drag force exerted on the medical device by blood and thus enhance transport of the medical device to the treatment site.

The drag member may be adapted to increase an engagement area with a fluid such as blood.

The invention is further directed to a system comprising a medical device, a controlling unit, and an imaging device. Preferably, the medical device is a medical device as described herein. The controlling unit is adapted to navigate the medical device to a target location in a vasculature, in particular a treatment site.

The system according to the invention may be particularly suited for moving a medical device in a vascular network. The medical device maybe a catheter device or a microrobot. The medical device may comprise a head section with a magnetic part and a back section with a controlling line. The medical device may be moved in the vascular network in order to treat or diagnose a patient. The system may comprise a magnetic actuator, a controlling unit and a controlling line driver. The controlling line may be attached to the controlling line driver. The controlling line driver, when said controlling line it attached to the controlling line driver, is adapted to hold, to retrieve and/or release a controlling line at different speeds. The magnetic actuator is adapted to generate a magnetic field at a predetermined location. Preferably, the magnetic field is predetermined. The magnetic field may exert a force on the medical device, in particular the magnetic part of the medical device, such as to pull the medical device in a predetermined direction. The controlling unit may be adapted to balance at least three forces applied on the medical device. Preferably, the controlling unit balances the forces in real time. Preferably, the three forces include at least one of a drag force acting on the medical device by a fluid flow, for example blood in a vessel, a force by the controlling line, and a magnetic force by the magnetic actuator. The controlling unit further operates the magnetic actuator and/or the controlling line driver.

The controlling unit can help the magnetic navigation. The concept described here helps to find a good equilibrium on the different forces applied on the medical device: flow force, gravity force, controlling force and the magnetic force or other potential forces acting on the medical device in order to navigate the medical device along the trajectory path. The system may automatically compute the forces and the relations between the forces and define the forces generated by the system, especially the controlling line force and the magnetic force, to ensure that the resulting force moves the medical device along a predefined trajectory.

This balancing model may allow also to optimize the distribution of forces induced by the magnetic actuator and the controlling line. The balancing of forces may also be beneficial for optimizing system requirements, such as e.g. lower magnetic fields and/or lower controlling line forces.

The controlling line driver may comprise, preferably consist of, any one of or a combination of a pulley, a linear actuator, a reel, an electric motor, a spindle, a gear, a screw and/or a nut, a linear gear track, and a continuous track. The controlling line driver may also comprise two or more of any one of these elements, also in combination with any one, two, or more of any other element.

The controlling line driver may also comprise, additionally or alternatively, a controlling line connector adapted for providing an operable connection between the controlling line driver and the controlling line.

Preferably, the controlling unit may comprise a processor and/or a memory. In a particularly preferred embodiment, the controlling unit is in operable connection with an electric motor and is adapted to control at least one of speed, power, and torque of the electric motor.

The speed may be at least partially predetermined, automatically determined or manually chosen. It is conceivable to use a combination of predetermined, automatically determined, and manually chosen speeds. For example, the controlling unit may calculate a suitable speed profile based on a planned trajectory in a vessel taking into account data about the flow of blood in said vessel and save the speed profile in a memory. Additionally or alternatively, the speed of the controlling line may be adapted during an intervention automatically, for example via feedback loop taking into account a planned trajectory and actual position data, and/or manually by a user. To this end, the system may preferably comprise an interface for a user, for example one or more touch screens, knobs, buttons, levers, adapted for allowing input of speed parameters. It is possible to use the same or additional interfaces for inputting further parameters related to control of position and speed of the medical device.

Preferably, the controlling unit is adapted to calculate a magnetic field at a device position in space and/or a force exerted on a magnetic element by said magnetic field when the magnetic element is positioned at the device position in space. The controlling unit may in particular take into account at least one of a position, an orientation, and/or a power of the magnetic actuator. Additionally or alternatively, the controlling unit may be adapted for receiving data from a sensor at or close to the device position, in particular data related to the magnetic field and/or force at the device position.

Additionally or alternatively, the device may calculate at least one of a position, an orientation, and a power of the magnetic actuator suitable to achieve a magnetic field and/or a magnetic force at the device position. The magnetic field and/or magnetic force may be calculated qualitatively (e.g. only a direction) or quantitatively.

Preferably, the controlling line driver is adapted to control at least two controlling lines attached the medical device. The two controlling lines may be released at the same velocity. Alternatively, the two controlling lines may be released at different velocities such as to position and/orient the medical device in the front of the thrombus.

The controlling line driver may comprise a sensor allowing to monitor the retrieving of the thrombus. As an example, a dynamometer is used to measure the force during the thrombus retrieving (entire procedure). The retrieving velocity of the thrombus may be regulated using a force measured by the controlling driver and/or the sensor.

The controlling unit may monitor physiological data measured by the system and/or data measured by other devices. The controlling unit may adapt the instructions to the different elements of the navigation system based on the monitored data.

The invention is further directed to a method of producing a medical device, in particular a medical device as disclosed herein. The method comprises the steps of
- providing a flexible line with a magnetic part and an attachment element attached thereto;
- coating at least a part of the attachment element with a temporary layer;
- coating at least a part, preferably the entire, of the medical device with a coating, preferably a resin and/or a polymer, wherein the coating step is preferably performed by dipping and/or drying;
- removing the temporary layer.

The method is particularly suitable if the attachment element comprises electric and/or electronic component, for example if the attachment element comprises electrodes for cauterization and/or electrothrombosis as described herein.

The method may further comprise a step of, prior to providing the temporary layer, coating the attachment layer with a coagulant and drying said coagulant.

The temporary layer protects the attachment element. The coating which is coated on at least a part of the medical device can be easily removed in areas that are protected with a temporary layer. Thus, the temporary layer prevents that the attachment element is coated with a coating. Thus, the method provides a particularly simple way of producing a medical device with a coating wherein a portion of the device is selectively not coated.

The invention is further directed to a method of retrieving a thrombus from a vessel using a medical device. Preferably, the medical device is a medical device as disclosed herein. The method comprises the steps of
- introducing a distal end of the medical device in a vessel, the distal end comprising an attachment element;
- navigating the medical device to a target location;
- Optionally, activating an activation mechanism such that the attachment element is transformed from a deactivated state to an activated state where it is attachable to the thrombus, preferably on a proximal side of the thrombus;
- attaching the attachment element to the thrombus on a proximal side of the thrombus;
- Optionally, stretching the thrombus to reduce the diameter of the thrombus in a cross-section perpendicular to a longitudinal axis of the vessel;
- Removing the thrombus from the target site by pulling on said thrombus.

Preferably, a retrieving velocity is adapted such as to control a force applied on the thrombus.

Preferably, the method further comprises a step of, after attachment of the attachment element to the thrombus, providing a vibration or shaking motion to the medical device to facilitate removal of the thrombus.

It will be understood that all method steps described herein in the context of medical devices and systems, in particular with respect to navigation in a vessel using the controlling unit, the controlling line and magnetic force, may be performed in the method according to the invention.

It will be understood, in the context of the present description, that proximal refers to a direction along the longitudinal axis of the medical device generally from the treatment side toward the medical device in its intended use, and distal from medical device toward the treatment site.

In general and in the context of the present description, a microrobot may generally be understood as a combination of a controlling line and a magnetic part. The microrobot may be a therapeuticmicrorobot further comprising a therapeutic tool.

In the following, the invention is described in detail with reference to the following figures, showing:
- Figs. 1a-1c:: A schematic representation of a device according to the invention in a vessel with a thrombus and its use for reaching and removing a thrombus.
- Fig. 2a-2c:: A schematic representation of the embodiments of a device according to the invention with different attachment elements.
- Figs. 3a-3e:: A detailed schematic representation of the mounting of an attachment element on a magnetic part of a device according to the invention.
- Figs. 4a-4b:.: A schematic representation of the activation of an embodiment of the invention.
- Fig. 5:: A detailed representation of the embodiment of Fig. 2a.
- Figs. 6a-6b:: A schematic representation of thrombus retrieval with the embodiment of Fig. 2a based on biological attachment.
- Figs. 7a-7b:: A schematic representation of an alternative embodiment for thrombus retrieval based on biological attachment using electro-thrombosis.
- Figs. 8a-8b:: A detailed view of the device shown in Fig. 7a. and 7b
- Fig. 9:: A different embodiment adapted for attachment using electro-thrombosis.
- Figs. 10a-10b:: A schematic representation of manufacturing steps for manufacturing the embodiment of Fig. 8a, 8b and 9.
- Figs. 11a - 11b:: A schematic representation of two embodiments for thrombus retrieval with a device based on chemical attachment.
- Figs. 12a-12c:: A schematic representation of different embodiments for mechanical attachment.
- Figs. 13a-13d:: A schematic representation of thrombus retrieval with a device based on mechanical attachment.
- Figs. 14a-14b:: A schematic representation of an embodiment with a spring.
- Fig. 15:: A detailed schematic representation of another embodiment based on mechanical attachment with an additional anchoring coating (thrombogenic, expansive material).
- Figs. 16a-16b:: A schematic representation of two alternative embodiments of a device with a protective coating.
- Fig. 17a-17b:: One embodiment for activation of protective coatings.
- Fig. 18a-18c:: Alternative embodiment for activation of protective coatings.
- Fig. 19a-19d:: Different embodiments of activation of an attachment element.
- Fig. 20a-20f:: Different embodiments of an attachment element.
- Fig. 21a-21f:: Schematically different attachment principles for devices.
- Fig. 22a-22b:: A schematic representation of a device with a protection frame.
- Fig. 23:: A schematic representation of a device with an additional propulsion element.
- Fig. 24a-24b:: A schematic representation of a device with a suction mechanism.
- Fig. 25:: A schematic representation of a device with mechanical grippers and a shaft.
- Fig. 26:: A schematic representation of a device with t-PA coating.
- Fig. 27:: An alternative device adapted for t-PA delivery.
- Fig. 28:: A schematic representation of a device with fibers.
- Fig. 29:: A cross-sectional representation of an alternative device with a suction mechanism.
- Fig. 30:: schematically a device comprising a balloon.
- Figs. 31a-31b:: schematically a first embodiment of a device with a sensor.
- Figs. 32a-32b:: schematically a second embodiment of a device with a sensor.

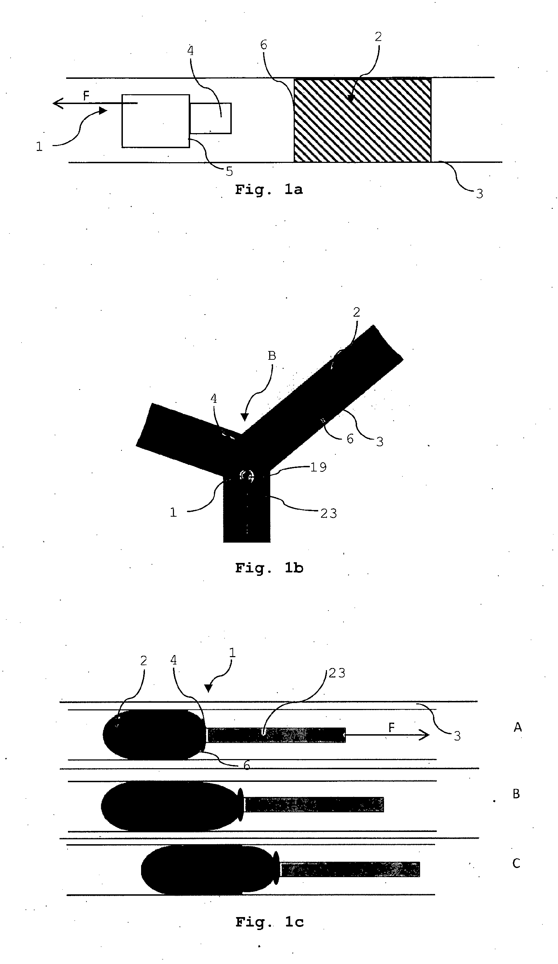

Figures 1a schematically shows a device 1 according to the invention and its use. The device 1 comprises an attachment element 4 which is adapted for providing attachment to a thrombus 2. It will be understood that the representation shown here is generic and intended for the reader's understanding of the basic principle of the invention, while specific embodiments will be shown thereafter. The device 1 may be guided to a treatment site, where the thrombus 2 is located by magnetic guiding and/or a catheter device (not shown). Once attached to the proximal side 6 of the thrombus 2, a force F can be exerted on the device 1 such as to pull the thrombus 2 from the treatment site in the direction of force F. The force F is thus sufficient to overcome a friction force between the thrombus 2 and the vessel wall 3.

Fig. 1b shows a device 1 being navigated to a treatment site where a thrombus 2 is located between vessel walls 3, thus, hindering blood flow. The device 1 comprises an attachment element 4 for attaching to said thrombus 2. Here, the device 1 comprises a magnetic part 19 which is attached to a controlling line 23. The controlling line 19 may be used to control the velocity in a blood stream. The magnetic part 23 may enable navigation by an magnetic field created by an external magnet. For example, an electromagnet and/or coils may be used. Here, the device has reached a bifurcation B. In order to reach the thrombus 2 at the treatment site, a magnet (not shown) may be used to guide the device 1 in the intended branch of the bifurcation B.

Fig. 1c schematically shows the removal of a thrombus 2 from a treatment site using a device 1 according to the invention. For clarity, reference signs for identical features are only shown once. Initially (panel A), the attachment element 4 is attached to the proximal side 6 of the thrombus 2. A force F is applied via the controlling line 23 in order to remove the thrombus 2.

In general, the device 1 may comprise a magnetic part 19 and a controlling line 23. The magnetic part can be used to control the trajectory of the device 1 in a blood vessel.

The magnetic part 19 can comprise or consist of soft ferromagnetic material such as iron, cobalt, nickel, hard ferromagnetic material such as Nd-Fe-B alloy, Fe-Pt alloys, ferrimagnetic material such as iron oxide. The magnetic part can comprise or consist of an aggregation of magnetic particles, particularly superparamagnetic nanoparticles made of iron oxide.
The magnetic part 19 may be coated to prevent direct contact with a biological fluid and thus avoid corrosion. For example, coating materials against corrosion can be polymers (such as Parylene C), ceramics (such as silica based, zirconium based, TiO₂) or metals (gold, silver).

The controlling line 23 may be used to control the displacement of the device 1. The controlling line 23 may be attached to a driver (not shown). The driver may be adapted to release the controlling line 23 according to instructions provided by a controlling unit (not shown).

The controlling line 23 may be used to slow down the velocity of the device 1 pushed by the blood flow, to stop the displacement and/or to move back the device 1. Additionally or alternatively, the controlling line 23 can be used to trigger a function in the device 1 by providing a stimuli.

The controlling line 23 can be connected reversibly or irreversibly to the magnetic part 19 by means of an adhesive. It is conceivable to additionally dip the magnetic part 19 and its connection to the controlling line into a resin. The resin may form a shell at least partially around the magnetic part 19 and a distal part of the controlling line 23, thus increasing the stability of the connection between the magnetic part 19 and the controlling line 19. Furthermore, improved adhesion the gluing, the glue can be functionalized with magnetic particles. The magnetic particles may be attracted to the magnetic part 19. Hence, the adhesion between the controlling line 23 and the magnetic part 19 can be icnreased due to magnetic interactions.

As shown in panel B, pulling of the thrombus by means of the force F exerted on the thrombus 2 leads to a stretching of the thrombus 2. Accordingly, a friction force between the thrombus 2 and the vessel wall 3 is reduced and less force is necessary to remove the thrombus 2 (see panel C).

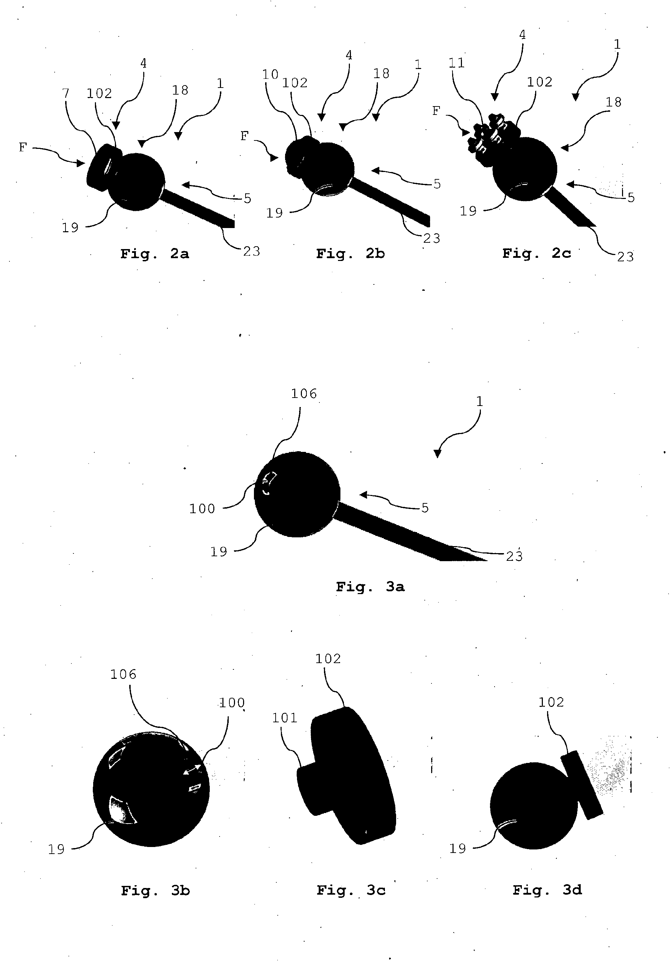

Fig. 2a shows one particular embodiment of a device 1 according to the invention. The device 1 comprises a magnetic head part 19 which is attached to a controlling line 23. The controlling line 23 is made of nylon with a diameter of 200 µm and a breaking force of 13 N. The attachment element shown here consists of a tool support 102 (see Figs. 3a-3d) arranged on the magnetic head part 19 and comprising a biological attachment element 7. The magnetic head part 19 is arranged at a distal end 5 of the medical device 1. The device 1 has, at a distal end, a substantially flat surface F.

Here, biological attachment refers to adhesion between a thrombus 2 and the device 1 by means a blood clot whose formation is induced by the biological attachment element 7. Thus, the biological attachment element 7 may be any thrombogenic substance.

Biological attachment elements 7 are particularly advantageous because a blood clot can be formed between the attachment element 4 and the thrombus 2 completely without or with a reduced need of a compression force to attach the device 1 to the proximal surface of the thrombus 2.

To trigger the blood clot formation, the biological attachment element may preferably comprise thrombogenic agents involved in the coagulation cascade, such as thrombin. The concentration of the thrombogenic agent in the biological attachment element may be chosen such that it is sufficient to trigger local, coagulation despite the presence of anticoagulant agents typically required during such interventions.

Here, the thrombogenic material which forms the biological attachment element 7 is attached to the surface of the surface of the tool support 102. The thrombogenic material may be chemically bonded to the surface of the tool support 102 by grafting.

Additionally or alternatively, the thrombogenic material may be mixed with a polymer, preferably a non-biodegradable polymer, to form a thrombogenic polymer which ensures a permanent attachment. The thrombogenic polymer may be dried on the surface of the tool support 102.

The thickness of the biological attachment element 7, in particular of the thrombogenic polymer coated onto the tool support 102, may be between 10 µm to 500 µm, preferably 90 to 110 µm.

Additionally or alternatively, the thrombogenic material may be mixed with a polymer solution, for example polyurethane with a solvent and then processed as fibers spun on the surface of the tool support 102 such as to form a biological attachment element 7. Preferably, hexafluorisopropanol (HFIP) is used as the solvent.

Additionally or alternatively, swelling polymers may be used. For example, hydrogels (PVA, PVP, Polyacrylamide), porous hydrogels, superabsorbent polymers (Poly(acrylic acid (PAA), foams (PU) may be used as a matrix material in a biological attachment element 4 to increase the contact surface with the thrombus after hydration by the blood.

If the biological attachment element 7 comprises fibers, said fibers may exhibit a core-shell structure. Preferably the thrombogenic agent is arranged in the shell. Preferably, a bioresorbable polymer forms the core and/or the shell.

The fiber diameter is preferably in the range of 50 nm to 10 µm, particularly preferably between 800 nm and 3 µm.

Particularly preferably, biodegradable polymers are used which exhibit a degradation time higher than two months. Polymers such as PLGA, PLLA, PDO, PCL may be suitable.

Alternatively, the biological attachment element may comprise an element adapted to create a reaction with a component of the thrombus, for example fibrin or red blood cells. Said reaction may trigger a coagulation cascade which will lead to the formation of a blood clot. For example, the biological attachment element may comprise peptides, in particular cyclic peptides such as Tn6, Tn7 and Tn10, which may react with fibrin in a thrombus 2.

Fig. 2b shows an alternative embodiment which is similar to the embodiment shown in Fig. 2a. The device 1 is formed as a microrobot 18 and comprises a magnetic head portion 19 connected to a controlling line 23 at a distal end 5 of the device 1. An attachment element 4 comprises a tool support 102. Instead of a biological attachment element, the tool support comprises a chemical attachment element 10. The device 1 has, at a distal end, a substantially flat surface F.

The chemical attachment element 10 may provide an adhesive force to a proximal surface of the thrombus 2. A compression force acting on the adhesive and compressing the adhesive on the thrombus 2 may be necessary to create sufficient adhesion. The compression force may be between 0.0.01 N to 3 N, preferably be-' tween 0.02 to 0.08 N. Preferably, the compression force is applied at least during 30 s, particularly preferably between 45 s and 120 s. The compression force can be achieved with a magnetic force generated by an external magnetic field and acting on the magnetic head portion 19.

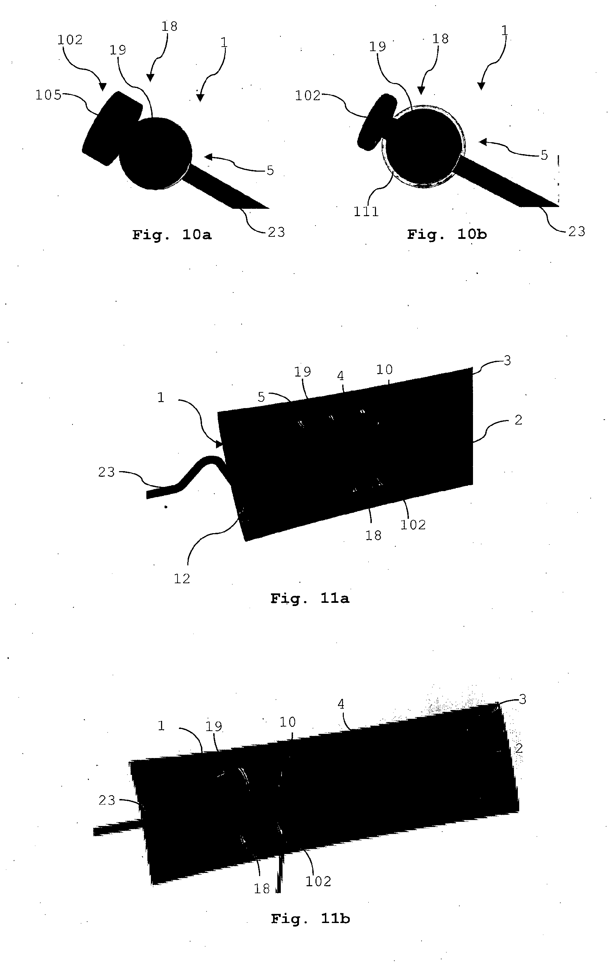

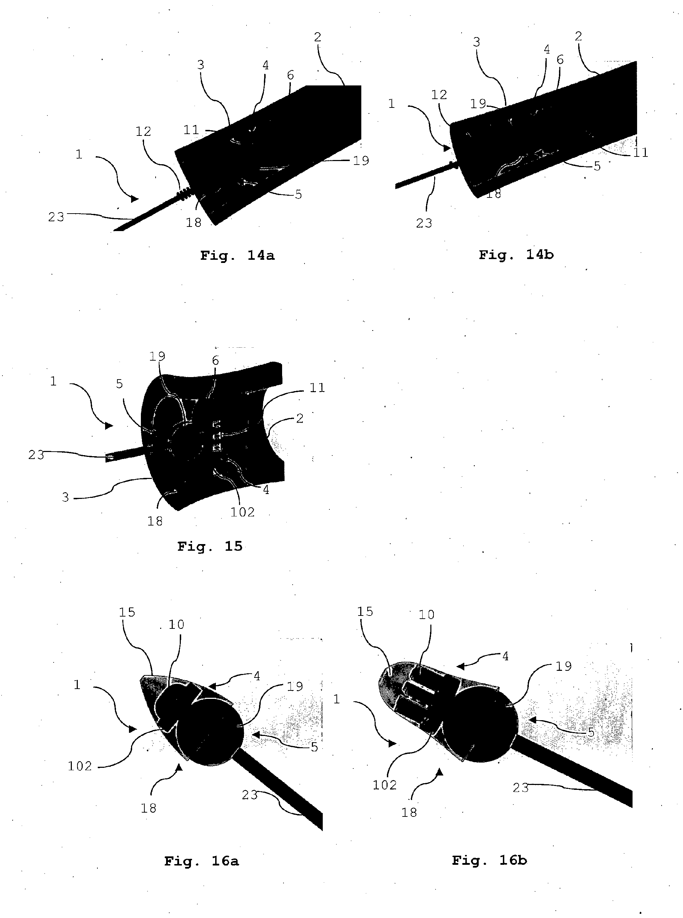

Additionally or alternatively, the compression force can be achieved by mechanical means. For example, a spring can be setup within the controlling line 23 (see Fig. 11a and 11b, and analogously Figs. 14a and 14b). The spring energy can be released once the device 1 is at an appropriate position with respect to the thrombus 2 for attachment.

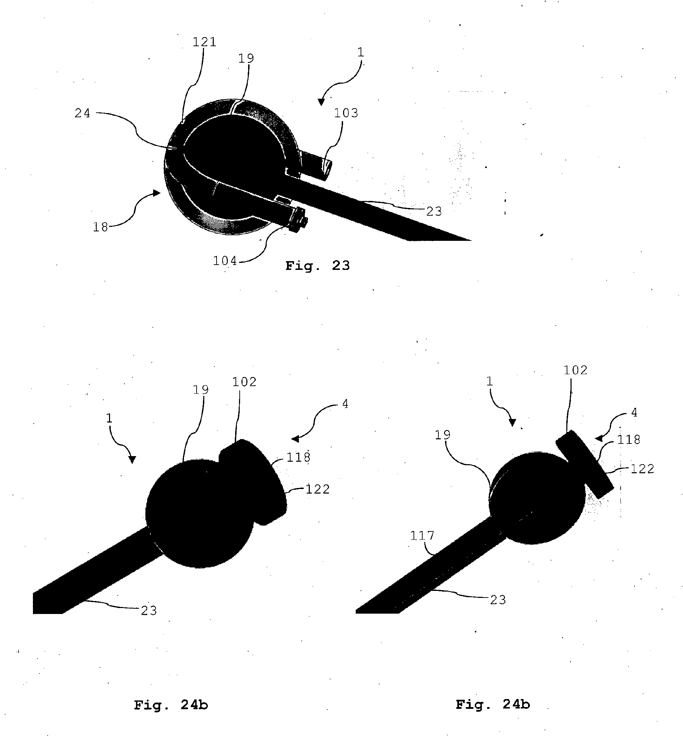

Additionally or alternatively, a propulsion element (see Fig. 23) may be used.

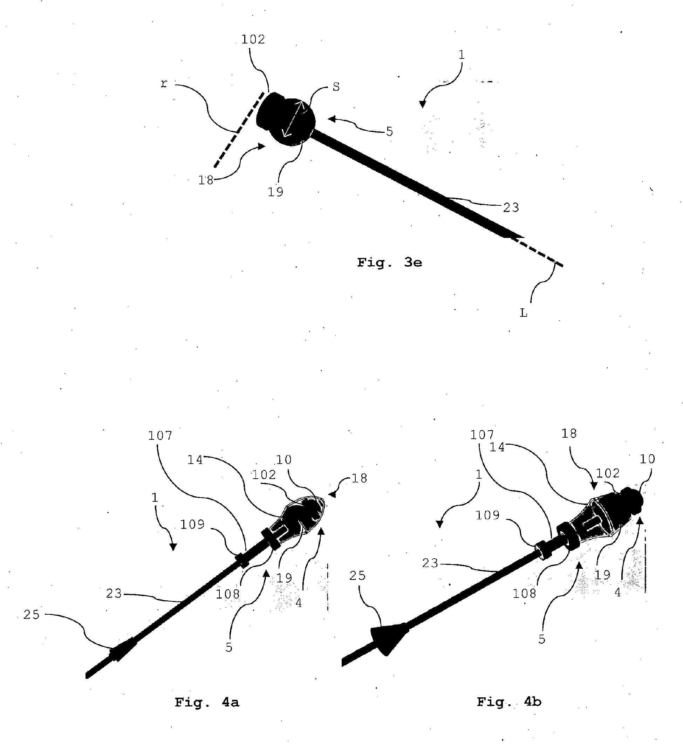

Additionally or alternatively, an drag element (see Fig. 4a and 4b), for example arranged on the controlling line 23 may be activated to compress the adhesive onto the thrombus surface 6.

Additionally or alternatively, any other means known in the art may be employed.

The adhesive material may be a n-Butyl-2-cynoacrylate such as 2-octyl-2-cyanoacrylate. The adhesive is a medical grade adhesive and may be arranged onto the anchoring support. Typically, the adhesive force of the adhesive is sufficient to hold the adhesive on the device 1, preferably the tool support 102. However, it is also conceivable to use adhesive enhancers such as an additional surface coating or the like.

Additionally or alternatively, the adhesive material may be biological. For example, the adhesive material may be a proteinbased (fibrin-based, collagen-based, gelatin-based, albuminbased) and/or polysaccharide-based adhesive (chitosan-based, alginate-based (e.g., SealG), and chondroitin-based).

Additionally or alternatively, the adhesive may be synthetic, such as polycyanoacrylates (e.g. n-Butyl-2-cynoacrylate such as 2-octyl-2-cyanoacrylate, polyurethanes (e.g., TissuGlu^{®}), poly (ethylene glycol) (PEG, e.g., DuraSeal^{™}, FoacaSeal^{®}, CoSeal^{®}), polyesters, as well as hyperbranched and dendrimer polymers.

Additionally or alternatively, the adhesive may be biomimetic, such as mussel-inspired adhesives (e.g., dopamine-modified gelatin + Fe3+ + genipin), gecko-inspired adhesives.

Additionally or alternatively, the adhesive might be present in an inactive state, and activated via any means known in the art and/or described herein, such as light activation (UV or other wavelength), or in situ mixing of at least two components. An example of light-activated glue may be a photo-polymerizable PEG glue, or a methacrylated tropoelastin. An example of in situ mixing may be a mix of two PEG polymers (such as in CoSeal^{®}), or of a PEG polymer and a poly(ethyleneimine) (such as in Adherus).

Additionally or alternatively, the adhesive may comprise magnetic nanoparticles, for example iron oxide nanoparticles. The nanoparticles may exhibit hard ferromagnetic, soft ferromagnetic, ferromagnetic, or superparamagnetic properties. The nanoparticles may have a characteristic size, in particular a diameter, between 5 nm to 400 nm, preferably 10 nm to 50 nm. Additionally or alternatively, nanoparticles may have a characteristic volume in the range of 50 nm³ to 3.4·10⁷ nm³, preferably 400 nm³ to 5.10⁴ nm³.

The nanoparticles may be dispersed in the adhesive and interact with the adhesive via low-energy Van der Waals forces. Alternatively, the nanoparticles may be connected to the adhesive via covalent and/or ionic bonds. The magnetic nanoparticles may thus provide magnetic properties to the adhesive dispersion comprising said nanoparticles. Thus, magnetic forces between the adhesive dispersion and the magnetic head part may arise and provide more secure attachment of the adhesive to the device 1. These magnetic forces may reduce the risk of detachment of the adhesive from the tool support 102 and at least partially counteract the strain applied on the adhesive during the retrievement of the thrombus.

The adhesive is arranged on the device 1 such as to have a diameter between 50 µm to 1 mm, preferably between 300 µm and 600 µm.

The adhesive may be a dried adhesive. A dried adhesive has a reduced adhesive strength compared to its wetted state. Thus, a dried adhesive is particularly advantageous if a protective shell is used during navigation to the treatment site, as the risk of accidental and/or permanent adhesion of the attachment element to the protective shell and/or other parts of the device may be reduced.

Additionally or alternatively, the adhesive may be impregnated into a porous material arranged on the tool support 102. The porous material may be fibrous tissue or porous beads.

For example, a fibrous fabric made of PET may be used. The fibrous fabric may comprise fibers with a diameter of 3 µm and a pore diameter of about 800 nm. The fibrous tissue can be fixed on the tool support 102 by means of a glue layer at the center and/or on the perimeter of the PET fibrous fabric. Preferably, the adhesive may be dried on the porous material such as the fibrous fabric.

It is particularly advantageous to use a fibrous fabric which is glued at a center as a circumferential area may remain unattached to the tool support 102 and thus provide increased attachment by moving toward the thrombus 2.

Fig. 2c shows schematically an embodiment of a device 1 wherein the attachment element 4 comprises mechanical attachment means 11. The embodiment shown here is, with the exception of the means of attachment, substantially similar to the embodiments shown in Figs. 2a and 2b. The device 1 has, at a distal end, a substantially flat surface F.

Here, the mechanical attachment element 11 is adapted to penetrate the proximal surface (see Fig. 1) of the thrombus 2. Optionally, the mechanical attachment element may comprise a thrombogenic coating (not shown), in particular a coating which is adapted to react with components of the thrombus to trigger coagulation. Thus, it will be understood that such an embodiment may thus combine mechanical and biological attachment means. Additionally or alternatively, the mechanical attachment element may be coated with an adhesive and/or an expansive material as described herein.

Expansive materials may generally be used to improve attachment to the thrombus.

Here, four mechanical attachment elements 11 are present. It will be understood that any number of elements 11 may be used depending on the intended application, in particular the size of the thrombus 2 to be treated. Preferably, between two and eight elements 11 are used. It is also possible to use exactly one element 11.

The mechanical attachment elements 11 have a higher Young's modulus than the thrombus 2 and is preferably at least above 1 MPa.

The diameter of the mechanical attachment element 11 here is 100 µm each, but may be in a range of 50 µm to 700 µm, preferably 80 µm to 300 µm.

The mechanical attachment element 11 preferably comprises or consists of a polymer, such as PET, PE or PP, or any blends or co-polymers thereof.

If mechanical and biological attachment means are combined, a thrombogenic element may be mixed with said polymer material. Thus, the mechanical attachment elements 11 may be made of a thrombogenic material.

Additionally or alternatively, the mechanical attachment element can comprise or consist of a metals. For example, stainless steel (316 L), titanium, tungsten, or any alloy thereof may be used.

Additionally or alternatively, a thrombogenic agent can be coated onto the mechanical attachment elements 11, for example by dip coating. Coating is advantageous in that it can be used with a wider variety of materials, i.e. is suitable with mechanical attachment elements comprising polymers, metals, or any other material.

In particular, the surface of the mechanical attachment elements 11 may be coated with a thrombogenic agent by means of CVD, plasma spraying, or physical vapor deposition.

Exemplary thrombogenic elements which may be employed are Von Wilerbrand factor (vWF), laminin, thrombospondin, vitronectin, fibrinogen or Thromboxane A2. These thrombogenic elements may trigger platelet adhesion and thus clot formation.

Thrombin is also suitable and is an enzyme which converts fibrinogen, by cleavage of fibrinopeptide A, into fibrin. Thus a fibrin network may be formed to capture erythrocytes and platelets leading to formation of a blood clot.

Figs. 3a to 3e shown in more detail the assembly of the device 1, in particular of the magnetic part 19 and the attachment element 4.

Fig. 3a shows the magnetic part 19 attached to the controlling line 12 on a distal part 5 of the device. The magnetic part 19 comprises an opening 100 which serves as a form-fit connection for a tool support (see Figs. 3c and 3d).

The diameter of the opening 100 is 300 µm in the shown embodiment. However, it will be understood that the diameter may be anywhere in the range of 50 to 600 µm, preferably 200 µm to 400 µm.

A height or depth 106 of the opening 100 can be equal or smaller to the diameter of the magnetic part 19. The height may preferably be between 100 to 1000 µm, particularly preferably between 200 µm to 400 µm.

Figure 3b schematically shows the magnetic part 19 of the device. The magnetic part 19 and its opening 100, which is arranged on a distal-most surface of the magnetic part 19, for fixation of the tool support (see Figs. 3c and 3d) are visible in greater detail. For clarity, the controlling line is not shown in the present figure. It will be understood, however, that the magnetic part 19 shown here may be connected to any controlling line 23 disclosed herein.

The magnetic part 19 shown here has a substantially spherical shape. Alternatively, the magnetic part 19 may have any other shape, in particular cubic, flat, elliptic or rod-like. The diameter of the magneticpart 19 here is 1500 µm, but may be in the range of 50 µm to 2000 µm, preferably between 200 µm to 1000 µm.

Fig. 3c shows the tool support 102 in greater detail. The tool support 102 is adapted to be setup at the distal end of the magnetic part 19.

The tool support 102 may be connected to the magnetic part 19 by gluing, screwing, clipping, welding or melting, for example. Preferably, the tool support 102 comprises a pin 101 intended for being arranged in the form-fit opening 100 of the magnetic part 19. It will be understood that any of the above connection methods may be combined with such a form-fit connection, i.e. an glue, a screw, a clip, a welded connection or a molten connection may be configured in addition to the form-fit connection.

Preferably, the tool support exhibits a flat surface. A characteristic size of the tool support 102, in particular the diamemter, may be equal, higher or lower than the diameter of the magnetic part.

The tool support may comprise or consist of a magnetic material, a non-magnetic material, or any combination thereof. For example, a part of the tool support 102 may be magnetic to improve its connection to the magnetic part 19.

Suitable non-magnetic materials are:
- metals, such as titanium, stainless steel, gold, silver,
- polymers, such as silicon, PEEK, polyurethanes;
- ceramics;
- composites.

The tool support 102 may, additionally or alternatively, comprise or consist of agglomerated particles. Agglomerated particles can provide enhanced tuneabity of material properties, for example the conductivity. The tool support 102 may optionally be coated with a biocompatible coating (not shown) to improve its biocompatibility.

Fig. 3d shows the magnetic part 19 of Fig. 3b assembled with the tool support 102 of Fig. 3c.

Fig. 3e shows an assembled device 1 configured as a microrobot 18. The device 1 comprises a magnetic part 19 as shown in Fig. 3b, a tool support 102 as shown in Fig. 3c, and is connected to a controlling line 23 at the'distal end 5 of the device 1. Here, no attachment element is shown on the. tool support 102, but it will be understood that any attachment element as disclosed herein may be combined with the shown embodiment.

Here, illustratively, a longitudinal axis L and a direction r perpendicular to the longitudinal axis is shown. The device 1 has a size S in a direction r which represents the maximum size of the device 1 in the direction r of 2 mm.

Figs. 4a and 4b show an embodiment of a device 1 which comprises and additional drag element 25. The device 1 shown here is substantially similar to the device 1 of Fig. 2b and comprises a magnetic part 19 with a tool support 102 which is configured with a chemical attachment element 10, here in the form of the drop of cyanoacrylate adhesive. The device 1 is configured as a microrobot 18. In addition, the device shown here comprises a protection cap 14 which consists of a capsule of thin foil of PVDF. Additionally or alternatively, PMMA and/or PA66 may be used. The protection cap 14 comprises a ring 108 which is slidably arranged on a slider 107. The protective cap 14 is retractable from the microrobot 18 by moving the ring 108 on the slider 107 in a proximal direction. The retractation can cause tearing of the protective cap 14 leading to its opening.

The protective cap 14 is crimped on the ring 108. The ring 108 may comprise or consist of a polymer, a metal, or any combination thereof. Crimping may prevent unintended penetration of biological fluids, such as blood, into the protective cap 14.

The ring 108 and the slider 107, which together form a sliding system, are setup on the controlling line 23.

The slider 107 may be configured as a configuration a straight tube, optionally with a distal ring (not shown) and a proximal ring 109. The straight tube allows the retractation of the capsule by providing a rail-like guide for the ring 108. Initially, the ring 108 is positioned at a distal end of the slider 107. To retract the protective cap 14, the ring 108 is moved to a proximal end of the sliding system. The ring may, for example, be moved by a line pulled via a controlling line driver (not shown). Alternatively, the crimping ring may be moved by contraction of a rigid line arranged between the ring 108 and the distal ring. The rigid line may, for example, comprise or consist of nitinol compressed under an electrical stimulus.

To avoid penetration of a liquid, for example blood, between the controlling line and the sliding system, a valve may be arranged inside the proximal ring 109.

Optionally, to facilitate the'breaking of the protective cap 14, the protective cap may comprise a predetermined breaking point, for example in the form of a reduction of the thickness (not shown). The reduction of the thickness can be between 10 to 70 %, preferably 30% to 50%. Additionally or alternatively, the breakable part can comprise or consist of a bioresorbable material, in particular a bioresorbable material as described herein. The degradation may be triggerable with any stimulus as described herein.

Alternatively, the tool support 102 may comprise a cutting tool (not shown). The cutting tool may tear the protective coating 14 during the retraction.

Preferably, the cutting tool is arranged on an edge of the tool support 102.

The drag element 25 is arranged on the controlling line 23. In one configuration, the activable drag element 25 is made of a nitinol frame covered with a polymer layer. The polymer layer may comprise or consist of polyurethane and exhibit a thickness between 50 µm to 300 µm. The nitinol frame can be activated under a stimulus such as heat leading to the opening of the structure. The increased drag caused by the blood flow can provide an additional propulsion force.

Fig. 4a shows a first configuration of the device 1. Here, the protective cap 14 is in a delivery configuration and protects the attachment element 4 from contact with blood, other biological fluids, and tissue. The drag element 25 is in a closed configuration.

Fig. 4b shows a second configuration of the device 1. Here, the protective cap 14 was opened by sliding of the ring 108 on the slider 107 in a proximal direction. The protective cap 14 thus tore and a distal-most end of the device 1, thus releasing the attachment element 4 from the protective cap 14 such that a thrombus (not shown) may be attached to the adhesive attachment element 10. In addition, the drag element 15 is in an open configuration and creates an additional drag force due to the blood flow.

It will be understood that the two configurations shown in Figs. 4a and 4b, i.e. wherein the drag element is closed during delivery and open when the attachment element 4 is released, are exemplary. It would be conceivable to open and/or close the drag element 25 independently of the configuration of the protective cap.14 (i.e. during delivery or during attachment of the attachment element to the thrombus, the drag element 25 may be open or closed). In particular, the drag element 25 may be used for additional drag during delivery (i.e. when the protective cap 14 is intact) and/or to provide an additional attachment force (i.e. when the attachment element 4 is released). It would also be possible to only open the drag element 25 during the delivery but not during attachment.

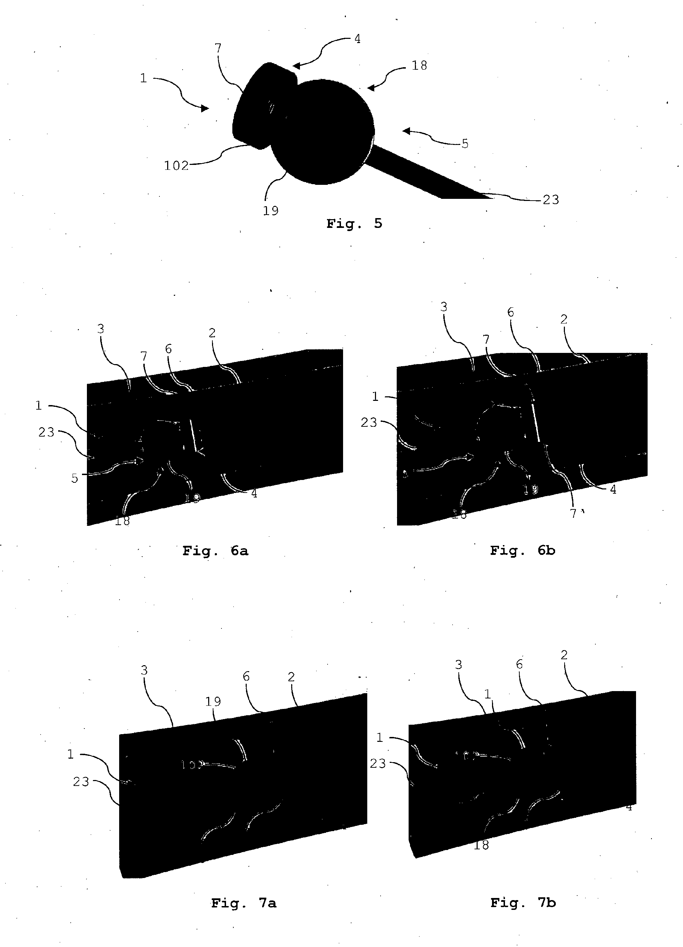

Fig. 5 shows a more detailed view of the distal end area of a device 1 similar to the device of Fig. 2a. The biological attachment element 7 comprises a porous polymer layer with a thrombogenic element arranged therein. The biological attachment element is attached as a thin layer on a tool support 102, which is attached to the magnetic part 19. A controlling line 23, formed of a thin nylon fiber, is attached to the magnetic part 19 and thus arranged at the distal end 5 of the device 1.

Figs. 6a and 6b show schematically the attachment of a biological attachment element 7 to a proximal face 6 of a thrombus 2. The device 1 shown here is substantially similar to the device shown in Figs. 2a and 5.

Fig. 6a shows the device upon delivery to a position in relative proximity of the thrombus 2. The biological attachment element 7 is oriented towards the proximal face 6 of the thrombus 2. In this position, the biological attachment element 7 may be activated. Here, an electrical signal is transmitted through a cable in the controlling line which releases a thrombogenic agent. It will be understood that more than one cable may be used. It will be understood that any activation mechanism disclosed herein, in particular a protective cap (see Figs. 4a and 4b) may be used in addition or as an alternative. It will be noted that the device moved up close the proximal face 6 of the thrombus, but is not in direct contact with the proximal face 6.

The shown embodiment in particular allows to trigger the formation of a thrombus by applying electricity (electro-thrombosis) .

Fig. 6b shows the device 1 of Fig. 6a after activation of the biological attachment element 7. A blood clot 7' has formed between the biological attachment element 7 and the proximal face 6 of the thrombus 2 due to the thrombogenenic activity of the biological attachment element. The blood clot 7' provides attachment between the thrombus 2 and the device 1.

As shown in Figs. 6a and 6b, a biological attachment element 7 may be advantageous in that the blood clot 7' is formed and may fill the entire gap between the device 1 and the thrombus, such as to provide attachment, even the alignment between the attachment element 4 and the thrombus 2 is imperfect. Thus, treatment may be easier to perform for a surgeon, in particular if the relative positions of the thrombus 2 and the device are not known with sufficient precision and/or if the proximal surface 6 of the thrombus 2 is shaped irregularly.

Figs. 7a and 7b show schematically an alternative strategy to achieve a biological anchoring using an electrical current applied via the tool support 102. It will be appreciated that the device which may be used for the method shown here will be described in more detail below. Here, for clarity, the working principle is described.

An electric current can lead to the formation of a blood clot by electro-thrombosis. Thus, similar attachment as, for example, shown in Figs. 6a and 6b may be achieved using an electrical current.

Thus, an electrical current can be delivered to the tool support 102 in order to achieve biological attachment to the thrombus 2.

Additionally or alternatively, an electrical current may also increase the temperature of the tool support 102, and thus lead to attachment of the tool support 102 by cauterization. The temperature can be increased up to 60°C, preferably to a temperature between 40 to 45°C. It is also possible to use a cryogenic probe at or on the tool support for cauterization. Heating of the tool support may be provided by an internal stimulus or an external stimulus.

Fig. 7a shows the tool support 102, which is attached to the magnetic part 19 of the device 1, in contact with the proximal surface of the thrombus 2 after delivery, but before activation of the attachment mechanism. The person skilled in the art will understand that the tool support 102 may also penetrate the surface of the thrombus 2 before activation. Additionally or alternatively, heating may be combined with the shown embodiment in order to further provide adhesion.

Fig. 7b shows the formation of formation of cauterized tissue 7' in the thrombus 7' after delivery of an electrical current and/or heat via the tool support 102. The cauterized tissue 7' provides sufficient attachment between the thrombus 2 and the tool support 102 in order to retrieve the thrombus.

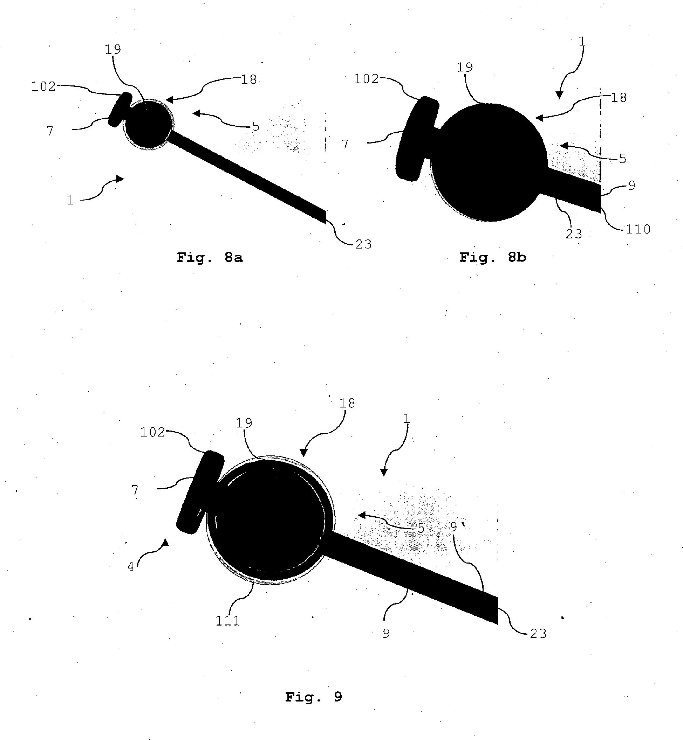

Fig. 8a shows schematically a device 1, configured as a microrobot 18, according to the invention comprising a biological attachment mechanism 7 which is adapted for inducing electrothrombosis. A electrical wire (not shown, see Fig. 8b) is arranged within the controlling line 23. The controlling line 23 is attached to a magnetic part 19 at a distal end 5 of the device 1. A tool support 102 is attached to the magnetic part 19 and adapted to the deliver an electrical current such as to act as a biological attachment element 7.

Fig. 8b shows a more detailed view of a distal area 5 of the device 1 of Fig. 8a in a crosssectional view. Here, an electrical wires 9 are embedded in an inner area of the controlling line 23 and connected to the magnetic part 19. For clarity, only one wire is shown, but the person skilled in the art will understand that two or more wires 9 may be present. The current can be delivered through the magnetic part 19 to reach the tool support 102. The diameter of each electric wire 9 is between 10 µm to 500 µm, preferentially 50 µm to 100 µm. The electric wires 9 may be attached to the magnetic part 19 by welding, melting or gluing with a conductive glue.

An insulator shell 110 is arranged on an outer surface of the controlling line 23 in order to avoid dissipation of electrical current in the biological fluids, in particular the blood. The insulator shell 110 is preferably made of a flexible polymer with a low Young modulus. The Young's modulus is preferably between 0.2 GPA and 50 GPa, particularly preferably between 0.5. GPa and 5 GPa. The thickness of the shell is between 20 to 200 µm, preferably between 30 to 80 µm.

Fig. 9 shows an alternative embodiment of a device 1 which comprises a tool support 102 configured as a biological attachment element 7.

Here, the two electrical wires 9, 9' are guided through the controlling line 23 and around the magnetic part 19 and connected directly to the tool support 102. The connection between the electrical wires 9, 9' and the tool support 102 is welded in this embodiment. The diameter of the electric wire is between 10 µm to 200 µm, preferably between 60 µm to 120 µm. The Young modulus of these wires is between 0.2 to 50 GPa, preferably between 0.5 GPa and 5 GPa. In the shown embodiment, the wires. 9,9' are arranged at a circumference of the magnetic part 19 and thus increase the diameter of the magnetic part. The described diameters of the wires 9,9'' are generally advantageous in such embodiments as navigation in blood vessels may be more challenging with increasing size of the magnetic part 19.

It will be understood that the electric wires 9,9' shown here may be used in any other embodiment disclosed herein to deliver an electrical current/voltage.

Furthermore, in order to prevent dissipation of current into the magnetic part 19, the magnetic part 19 is coated with a protective shell 111. The protective shell 111 is electrically insulating and may provide additional protection against corrosion. The protective shell 111 here consists of a layer of polyester resin with a thickness of 100 µm. Additionally or alternatively, the protective shell 111 may, however, comprise any ceramic, polymer or a composite material. For example, epoxy resins, silicones, or epoxy-polyurethane blends are suitable materials. Carbon, silica-based ceramics, zirconium nitride or zirconia are suitable materials as well. The thickness of the protective shell 111 may be in the range of 50 nm to 200 µm, preferably between 30 µm to 100 µm.

The tool support 102 may be used as a probe for the electrothrombosis or cauterization. It will be understood that the shown shape is exemplary and that other shapes may be employed.

Figs. 10a and 10b schematically show a method of producing device 1 according to the invention. The method shown here is particularly suitable to produce a device 1 as shown in Fig. 9.

Fig. 10a shows a device 1 wherein the magnetic part 19 has been assembled with the controlling line 23. The tool support 102 is connected to the magnetic part 19 substantially in the way disclosed herein. Here, a temporary layer 105 is arranged on the tool support 102. The temporary layer 105 comprises a polyvinylalcohol (PVA) and has a thickness of 100 µm. The thickness may be any value in the range of 50 µm to 200 µm. The temporary layer 105 covers the tool support 102 entirely in the shown example, but does not extend beyond said tool support 102. As such, the magnetic part 19 and the controlling line 23 are not covered by the temporary layer. A protective shell (not shown, see Fig. 10b) can be coated onto the device 1 including the temporary layer 105.

Alternatively, the temporary layer 102 may be a silicone cover which may be stretched and placed over the tool support 102. The silicone cover preferably has a thickness of 100 µm to 300 µm.

Fig. 10b shows the device 1 after finalization of the manufacturing of the device 1. As described in the context of Fig. 10a, the protective shell 111 was coated onto the device 1 while the temporary layer 105 was still present. The protective shell can be cut in the general area of the tool support 102. Subsequently, the temporary layer 105 (see Fig. 10a) is removed by dissolution in water. As a result, the protective shell 111 is exclusively arranged on the magnetic part 19 (where a temporary layer way present, the protective shell 111 is removed together with the temporary layer 105). The tool support 102 is not covered by the protective shell 111. Here, the device 1 is substantially similar to the device 1 shown in Fig. 9 and the tool support 102 is adapted for delivering an electrical current. The lack of protective shell 111 reduces the overall resistance and thus facilitates delivery of electrical current.

Alternatively, where a silicone cap as described above is used, said cap is cut and removed from the tool support 102 such as to remove the protective shell 111.

The method as described above allows for an simple and less error-prone way of selectively coating certain parts of the medical device with a protective shell, but not others. In particular, other methods (etching or scratching the protective coating) may damage the device and increase scrap rates.

It will be understood that the example above is exemplary in nature and other parts of the medical device may be covered by a temporary layer in. addition or as an alternative.

Fig. 11a shows an embodiment of a device 1 comprising a chemical attachment element 10 during a treatment wherein a thrombus 2 is retrieved from a vessel 3. The device shown here is similar to the device shown in Fig. 2b and is configured as a microrobot 18 comprising a magnetic part 19, a tool support 102 with an adhesive 10 arranged thereon, and a controlling line 23 attached at a distal end 5 of the device 1. Here, the device additionally comprises an extendable element 12 configured as a spring. The spring 12 is adapted to provide a compression force of 0.1 N onto the thrombus 2 in order to attach the adhesive 10 to the thrombus. The compression force may be in the range of 0.05 N to 2 N.

Fig. 11b shows a similar treatment step as shown in Fig. 11a. However, the device shown here does not comprise a spring. Instead, a surgeon may use a magnetic field to provide a force in a distal direction on the magnetic element in order to attach the adhesive attachment element 10.

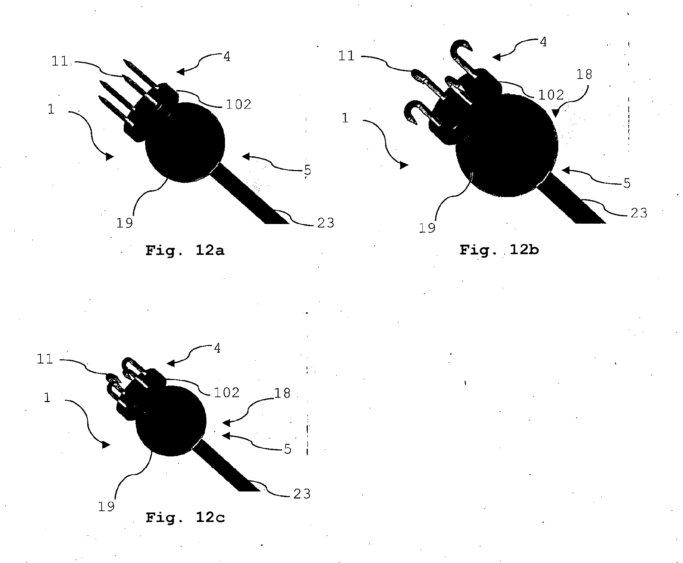

Fig. 12a shows a detailed view of a device 1 with a magnetic part 19 and a controlling line, wherein mechanical attachment elements 11 are attached to the tool support 102 which is arranged at a distal end of the magnetic part 19. It will be understood that the controlling line 23 and magnetic part 19 are substantially similar as described herein. The tool support 102 is glued to the magnetic element. Four mechanical attachment elements 11 are arranged on the tool support 102 by a screwing mechanism (not visible) and comprise a shape memory alloy. Additionally or alternatively, a form fit and/or adhesive may also be used. The mechanical attachment elements 11 have a diameter of 60 µm and a length of 0.5 mm. The diameter may be in the range of 0.3 mm and 0.8 mm. Alternatively, for catheter-based devices, the diameter may be between 1 mm and 3 mm. Here, the mechanical attachment elements 11 are arranged perpendicularly with respect to the surface of the tool support 102. However, any angle between 10° and 160° would be conceivable, and an angle between 60° and 120° is preferred.

Fig. 12b shows the device 1 of Fig. 12a wherein the mechanical attachment elements 11 are bent outwardly. The transition from the straight configuration to the bent configuration shown here can be triggered by a change in temperature, for example, which may be achieved by delivery of an electrical current.

In this design, the mechanical attachment elements 11 are based on rigid wires which can be bent according to any stimulus known in the art. For example, as described above, the wires, can be comprise or consist of nitinol which is bent due to an electrical current.

To avoid a complete bending of the wire, the wire may be setup inside a rigid shaft. The shaft can be made of a polymer (PEEK for example) or a metal (stainless steel AISI 316L, for example). The internal diameter of the shaft may be between 50 µm and 500 µm, preferably 100 µm to 300 µm. The external diameter of the shaft may be between 90 µm to 600 µm, preferably 140 µm to 350 µm.

As described above, the wires may be arranged on the tool support 102. The connection between wires and tool support may be through holes, or via a matrix which is fixed to the support. The diameter of the wires generally may be between 50 µm and 300 µm, preferably 75 µm and 150 µm. The extremity of the wire can be shaped in order to improve the penetration of the proximal surface, in particular have a pointy and/or sharp shape.

Fig. 12c shows a different embodiment wherein the mechanical attachment elements 11 bend inwardly instead of outwardly.

It will be understood that the features shown in Figs. 12b and 12c may be combined, for example by configuring some mechanical attachment elements to bend inwardly and some others to bend outwardly. Furthermore, it will be understood that the shape change shown here, wherein a section of the mechanical attachment elements 11 bend such as to form a hook-like shape is of exemplary nature. It would be conceivable to configure the mechanical attachment elements 11 such as to form any other suitable shape, such as a cork-screw shape, rings, kinks, and/or others.

Mechanical attachment generally refers to the attachment to the proximal surface of the thrombus using a mechanical system. It will be understood that the embodiments shown herein are of exemplary nature.

In general, mechanical systems are used to penetrate the thrombus. Penetration is preferably progressive at a rate between 0.01 mm/s to 15 mm/s, particularly preferably 0.1 mm/s to 1 mm/s, in order to avoid a damage to the thrombus structure. An intact thrombus is advantageous as it reduces the risk of breaking during retrieval or debris being released into the vessel system.

The number of mechanical attachment elements 11 arranged on the tool support 102 is generally between 1 to 20, preferably 2 to 8.

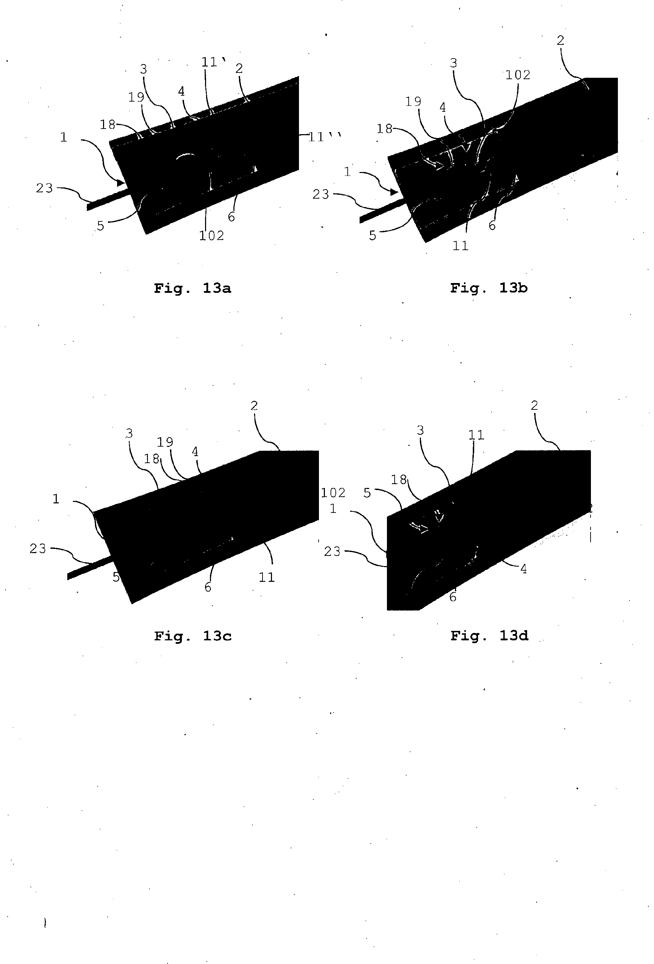

Figs. 13a-13d show a method of attachment of a thrombus 2 by means of an attachment element 4 comprising mechanical hooks 11. The device 1 shown here is similar to the device of Fig. 12b.

Fig. 13a shows the device 1 in proximity to the thrombus 2 which is intended to be retrieved. As mentioned, the device is substantially similar to the device described in Fig. 12b. In a delivery configuration, the mechanical attachment elements 11',11" are configured to be oriented in a bent shape pointed to the center of the tool support 102 in the absence of a stimulus/trigger mechanism. Here, as the device 1 has reached a general. area intended to be treated, a stimulus is used to change the shape of the mechanical attachment elements 11 into a straight shape. Illustratively, the device 1 here is shown in an state wherein one element 11' is still bent inwardly and another element 11" is already in a straight configurations.

The mechanical attachment elements 11', 11" are bent and may be separately activatable. To this end, each mechanical attachment element 11',11'' may be in connected or connectable to a separate electrical connection each. Such an arrangement may be advantageous when the thrombus has an irregular shape.

In order to provide separate activation to each mechanical attachment element 11', 11", the tool support 102 may comprise four parts glued together with an adhesive providing electrical insulation. Thus, current leakage through the glue is prevented. Each part of the tool support 102 may be connected with two electrical wires.

Fig. 13b shows the device 1 of Fig. 13a wherein the mechanical attachment elements are configured in a straight shape as a result of the completion of the shape change described in the context of Fig. 13a.

Fig. 13c shows the device of Figs. 13a and 13b after penetration of the thrombus 2. Here, a magnetic field force exerted on the magnetic part 19 by an external magnet (not shown), is used to propel the device1 in a distal direction such as to penetrate the thrombus 2 on its proximal face 6. In general, the penetration into the thrombus 2 may be achieved via any one of:
- a magnetic displacement controlled by the external magnetic field as described here, and/or
- a chemical propulsion (see Fig. 23), and/or
- a mechanical propulsion, for example achieved with a spring (see Figs. 11a), and/or
- a blood flow propulsion achieved with an additional drag element setup on the controlling line 23 (see Fig. 4a and 4b).

The penetration may monitored via the length released by the controlling line driver. Additionally or alternatively, a pressure sensor may be attached to the controlling line, in particular a pressure sensor in operable connection with the controlling line driver used to monitor the retrieval of the thrombus.

Additionally or alternatively, the penetration may be monitored by any imaging means known in the art. To this end, any part of the medical device, in particular the tool support and/or any attachment element, may be configured to be radiopaque and thus imaged when attached at or in a thrombus.

Fig. 13d shows the final attachment of the attachment element 4 with the thrombus 2. On the inside of the thrombus 2, the stimulus value can be increased in order to orient the mechanical attachment elements 11 to point outwardly and thus attach to the thrombus 2.

It will be understood that the specific shape change described here is of exemplary nature and any shape change that allows for penetration initially and attachment subsequently may be suitable. In particular, the attachment elements 11 may be bent inwardly instead of outwardly (see Figs. 12b and 12c).

Fig. 14a and 14b show schematically a method of attaching a thrombus using a mechanical attachment element 11 substantially similar to the method shown in Figs. 13a-13d.

Here, the device 1 additionally comprises a spring 12 incorporated into the controlling line 23. The spring 12 can provide a propulsion force to penetrate the thrombus 2 with the mechanical attachment elements 11. It will be understood that any other propulsion mechanism may be combined with the embodiment shown here, in particular magnetic propulsion as described in the context of Figs. 13a-13d.

Fig. 14a shows the device 1 in the general area intended to be treated, wherein the spring 12 is compressed and locked.

Fig. 14b shows the device 1 after the spring has been unlocked and released, thus providing a force in a distal direction sufficient to penetrate the thrombus 2 with the mechanical attachment elements 11.

Release of the spring 12 may be mechanically or electrically controlled. Mechanical release may be performed via a line attached to the spring 12. Application of an external force on the line may release the spring 12.

In particular, the spring 12 may comprise a shape memory material, for example a Nitinol alloy and may be triggered to change from a first shape to a second shape.

The spring 12 may be released by removing or degrading a physical barrier. The spring may in particular be made of a shape memory alloy, for example nitinol. Under a stimuli such as heat or electricity, the spring may be activated. To activate the spring by heat, it can be coated with a material which can be heated. For example, a spring may be coated with gold nanoparticles which can be heated when exposed to infrared irradiation. Another configuration can be a spring coated with superparamagnetic nanoparticles. These particles can be heated in an alternative magnetic field such as the one use for hyperthermia.
The physical barrier may be a lock. The lock can be setup with a frame parallel to the compressed spring. The lock is setup perpendicularly to the longitudinal direction. The lock may be removed by pulling or by fast degradation (biocorrosion of magnesium under a current). A part of the controlling 23 line may be formed by the spring 12, i.e a controlling line 23 may be set up between the controlling line driver (not shown) and the spring. 12 and a second portion of the controlling line 23 may be set up between the spring 12 and the magnetic part 19.

The spring may have a diameter comprised in the range of 50 µm to 800 µm, preferably 100 µm to 300 µm. In the extended state as shown in Fig. 14b, the spring may have a length between 1 mm and 30 mm, preferably between 2 mm and 5 mm. In a compressed state as shown in Fig. 14a, the spring 12 may have a length in the range of 0.5 mm to 10 mm.

Preferably, the spring 12 is adapted to change its length, when transitioning from the compressed to the extended state, by at least 100 %, preferably 200 %.

Preferably, the spring is adapted to apply a force between 0.05 N and 0.6 N.

The spring 12 may be formed by the controlling line 23. For ex-ample, the controlling line 23 may comprise a nitinol wire which is formed in a helical shape. Under a stimuli, for example electricity, the helical nitinol shape may be elongated. The diameter of the nitinol helix may be between 50 µm to 900 µm, preferably 100 µm to 300 µm.

It will be understood that the spring 12 shown here may be combined with any other attachment element as well, in particular a chemical attachment element comprising an adhesive (see Fig. 2b, for example).

Alternatively, the spring 12 may be configured as a separate element connected to the controlling line 23. Preferably, the spring is arranged between the magnetic part 19 and the controlling line 23.

Fig. 15 shows a device 1 substantially similar to the device of Fig. 2c when attached to a thrombus 2. Here, three mechanical attachment elements 11 are arranged in a line on the tool support. The attachment elements 11 have a minimum diameter of 120 µm and a maximum diameter of 200 µm towards a distal end of the element 11. The elements 11 have a length of 300 µm. The mechanical attachment elements are made of surgical-grade steel and are coated with calcium chloride in order to promote thrombosis, which additionally anchors the mechanical attachment elements 11 in the thrombus 2. Generally speaking, any adhesion mechanism (chemical or biological) may be used to provide additional anchoring after penetration. Preferably, the adhesion mechanism is arranged at a proximal area of the elements 11.

Optionally, the elements 11 may be coated with an expansive coating. Such a coating may provide additional anchoring to the thrombus 2 by expanding once the elements 11 are placed within the thrombus 2.

Fig. 16a shows an embodiment of a device 1 with a chemical attachment element 10 substantially as shown in Fig. 2b. A protection layer 15 is arranged on the attachment element 4 and partially on the magnetic part 19. The protective layer 15 comprises a polymer which is soluble in aqueous solutions and dispersions, such as blood. Preferred polymers are polysaccharides, cellulose, starch and/or starch-based copolymers and derivates, gelatin, hydroxypropyl methylcellulose (HPMC), gelatin-PEG, and/or PEO. The protective layer 15 is adapted, preferably in its thickness, such that complete dissolution in blood takes longer than a typical treatment time. Typically, dissolution will take place within 5-30 min, preferably 5-15 min. As a result, the device 1 can be navigated through a vessel system to a target site without the chemical attachment element 10 coming in contact with blood. However, once the target site is reached, the protective shell 15 will automatically dissolve within a short amount of time, for example 5 min, after which the chemical attachment element is released and can be attached to the thrombus.

Fig. 16b shows a similar device 1 as shown in Fig. 16b. A similar protective cap 15 is arranged over the attachment element 4. Here, the attachment element 4 comprises elongated adhesive elements 10 with a stiffness sufficient to penetrate a thrombus (not shown). The elements 10 may be metallic or polymeric elements with an adhesive coating, for example. Thus, once released, the chemical attachment element 10 can penetrate the proximal face of the thrombus, leading to increased adhesive strength and safer retrieval. Although not shown here, the elements 10 may be sharpened in order to improve penetration into a thrombus.

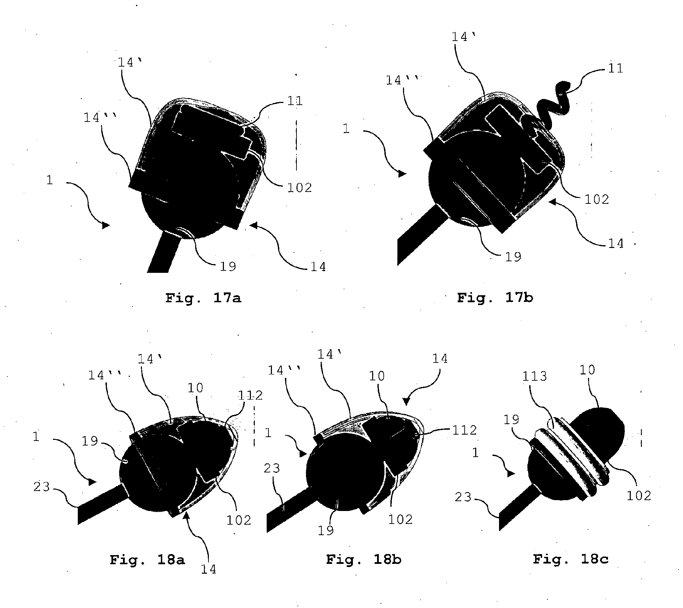

Fig. 17a and 17b show an embodiment of a device 1 with a mechanical attachment element 11 comprising a spring.

Fig. 17a shows the device 1 in a delivery configuration. The spring 11 is compressed onto the tool support 102 and has a substantially flat shape. A protective cap 14 is attached to the magnetic part 19 and prevents contact between the spring 11 and body fluids and tissue.

Fig. 17b shows the device 1 after an activation. Here, the spring 11 was used to break a film 14' which his part of the protective cap 14. By expanding the spring 11, a perforation of the film 14' is achieved.

Release of the spring 11 in order to perforate the protective cap 14 may be done by a locking mechanism that lets the spring 11 expand back to its original configuration when removed.

To maintain the spring 11 in a compressed configuration, nitinol parts can be used. By application of a current, the nitinol parts may be heated up and change their configuration and thus they may release the spring.

Here, the protective cap 14 comprises a film 14' and a ring 14". Preferably, the film 14' is made of a polymer such as PE. The film may be attached to the ring during its processing by dip coating. Additionally or alternatively, the film 14' can be glued, melted or welded on the ring 14". The thickness of the film 14' may be between 20 µm and 500 µm, preferably between 100 µm and 300 µm.

The ring 14" can comprise or consist of a polymer such as PEEK (polyetherkeytone), polysulfones (PSU, PPSU) or polyethylene (HDPE). Additionally or alternatively, the ring 14" may comprise a metal such as stainless steel (304 or 316L), titanium, tungsten.

The ring 14" may further comprise a magnetic material such as iron oxide, iron, Nd-Fe-B, FePt, thus giving the ring 14" magnetic properties. A magnetic ring can lead to magnetic interaction with the magnetic part 19 used for the navigation. This interaction improves the adhesion of the ring with the device 1.

The ring 14" may be coated to reduce or prevent corrosion.

The spring 11 may additionally be coated with a thrombogenic material or an adhesive material in order to provide further bio-logical or chemical attachment.

Aditionally or alternatively, a thrombogenic material may be arranged on the tool support 102.

It is also conceivable to use the spring solely for penetration of the protection cap 14, i.e. for activation of a separate attachment mechanism.

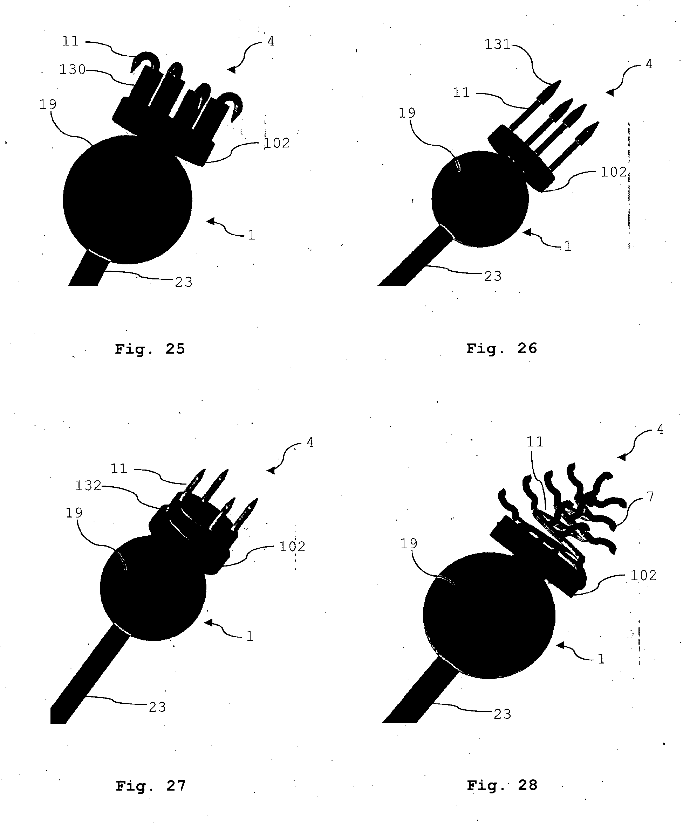

To this end, a thrombogenic material may be fixed to a scaffold which is attached to the spring 11 and/or to the spring 11 itself (see Fig. 28). The scaffold may be made of synthetic fibers. Synthetic fibers can be made of bioresorbable polymers such as polydioxanone (PDO) or poly-ε-caprolactone (PCL), or non-bioresorbable polymers such as PET or polyurethane. The scaffold may also comprise or consist of natural fibers, for example fibers of collagen. It is also conceivable to use a combination of natural and synthetic fibers. The thickness of the scaffold is between 50 µm to 2 mm, preferably between 150 µm to 300 µm.

In certain embodiments, a spring 11,12 may be setup between the magnetic part 19 and the tool support 102. The tool support 102 may comprise a cutting area in order to cut the protective cap 14 open. Preferably, four cutting areas are arranged on a circumferential area of the tool support.

Fig. 18a and 18b show an alternative embodiment of an activatable device in a delivery configuration. A protective cap 14 is similar as shown in Fig. 17a and comprises a polymer film 14' attached to a ring 14". The device 1 comprises a chemical attachment element 10 formed as an adhesive.

Fig. 18b shows the device 1 in a crosssectional view in a plane parallel to a longitudinal axis of the device 1. Here, the detailed construction of the device is visible, in particular the attachment of the tool support 102, magnetic part 19 and controlling line 23. In addition, an activatable breaking support 112 is visible and which is arranged within the chemical attachment element.

Here, in general, the polymer film 14" is arranged on the device 1 under tension. Thus, once broken distally, the polymer film 14" has a tendency to fold onto the ring 14'.

The thickness of the polymer film 14' may be between 20 µm and 700 µm, preferably 100 µm to 200 µm. The polymer preferably exhibits a breaking strain of less than 100%. The polymer layer may be attached to the ring 14'' in any way as described herein, in particular in the context of Figs. 17a and 17b.

In order to create a tension in the polymer film 14', the acti-vable breaking support 112 is setup on the tool support 102. The polymer layer 14' may be strained by the breaking support 112 while the ring is moved in a proximal direction until the ring 14'' reaches its intended position.

The activable breaking support 112 is adapted to change its shape by action of a stimulus. For example, the activable breaking support 112 may be made of nitinol. Thus, the activatable breaking support 112 may extend when subjected to an electrical current. As a result, further strain is applied to the protective cap 14, in particular the polymer film 14'.

Alternatively, the activable breaking support 112 may comprise a sharp element, such as a needle, which is arranged within the support 112 without contact to the polymer film 14', but movable to a position where it can be brought in contact with the film 14' such that the film is perforated. The sharp element may comprise or consist of a metal, such as stainless steel or titanium. The sharp element may have a diameter between 40 µm to 200 µm, preferably 70 µm to 150 µm. Additionally or alternatively, the sharp element may be adapted for anchoring to the thrombus.

Additionally or alternatively, the activatable breaking support 112 may be adapted to trigger a stimulus, for example an electric current, which can destabilize and thus break the polymer film 14'. The current, or heat caused by the current, may destabilize the polymer film 14'.

Electrical wires can be directly connected to the activable breaking support 112. Alternatively, the current can be provided via the tool support 102, in particular if the device is configured substantially similarly as shown in Fig. 9.

Fig. 18c further shows the expansion of a swelling material 113 arranged between the magnetic part 19 and the tool support 102. Here, the swelling material 113 is a swellable polymer and is adapted to provide a further compression force towards the thrombus (not shown). It will be understood that the swelling material 113 is shown here an exemplary embodiment, but is optional and not necessary in the context with the activatable breaking support 112. Furthermore, swelling materials as shown here may be combined with any other embodiment disclosed herein.

In general, swelling materials, in particular any of the polymers mentioned above, may be used to optimize the contact surface with the proximal surface of the thrombus. To this end, an attachment material such as the adhesive material or the thrombogenic material can be attached to the swelling material. The size of the material will increase under hydration. During the expansion, the adhesive material can cover the activable support.

Particularly suitable swelling materials are hydrogels (PVA, PVP, Polyacrylamide), porous hydrogels, superabsorbent polymers (Poly(acrylic acid (PAA), foams (PU).

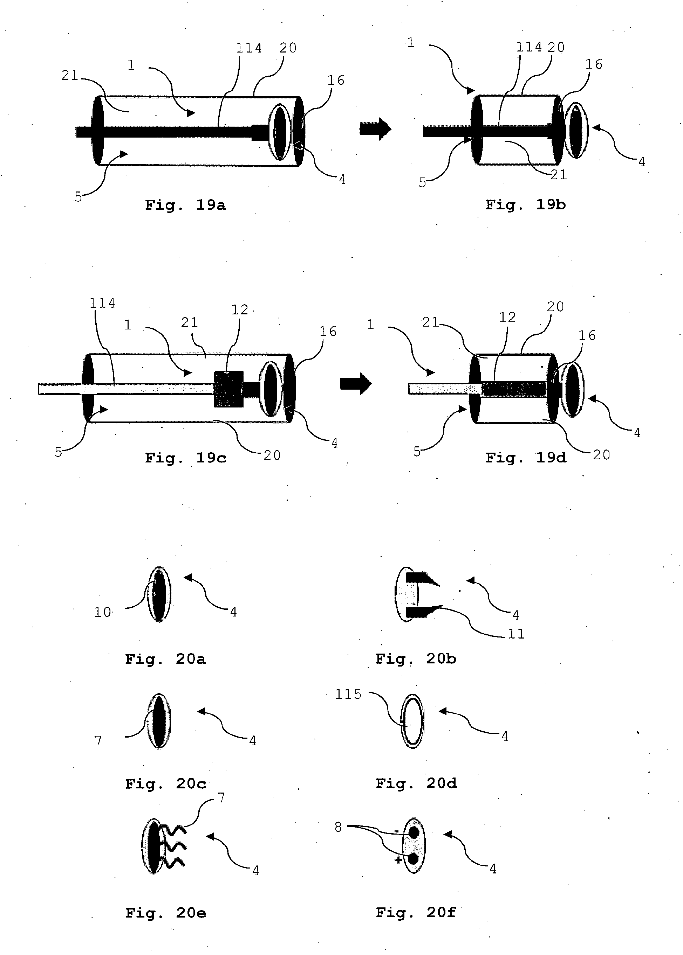

Fig. 19a-19d show different embodiments of devices 1 according to the invention formed by catheter devices 20. It will be understood that only the distal parts 5 of the catheter devices are shown for clarity.

Fig. 19a shows a catheter device 10 with storage compartment 21. Within the storage compartment 21 is arranged an attachment element 4 attached to a guiding wire 114. The guiding wire 114 has a sufficient stiffness to move the attachment element 4 back and forth and to provide a compression force. Here, the catheter device futher comprises a foil 16 made of a biocompatible polymer such as PE. The foil 16 seals the storage compartment 21 from the outside area and prevents entry of blood into the storage compartment 21.

As shown in Fig. 19b, by pushing the guidewire, the attachment element 4 can break the foil 16 and move to an outside position of the catheter 20. The attachment element 4 is thus activated and may attach to a thrombus.

Additionally or alternatively to pushing of the guidewire 114, injection of saline solution may be used to move the attachment element 4, in particular by activation of hydraulic means.

Additionally or alternatively, the attachment element 4, which is attached to a line and/or guidewire, may be flushed outside of the catheter device 10. The attachment element 4 may be moved into the catheter when the catheter is located at the thrombus or may be stored in a distal part of the catheter device 10 during delivery.

The attachment element 4 may be moved out of the catheter device 10 by means of a gas activation (e.g. air), liquid (e.g. saline solution), heat (e.g. electrical or induction) and/or electricity (e.g. piezoelectric material).

Fig. 19c shows an alternative embodiment of a catheter device 20.The arrangement of attachment element-4 within the compartment 21 of the catheter device 20 and the closure with a foil 16 is substantially similar to the catheter device of Figs. 19a and 19b. Here, an expandable element 12 is further arranged between the guidewire and the attachment element 4. The expandable element 12 may, for example, be a spring, and is fixedly attached to the catheter 20 with respect to the storage compartment 21.

As shown in Fig. 19d, the attachment element 4 may be moved outside of the catheter 20, breaking the foil 16 in the process, by expanding of the expandable element 12 without the need to push the guidewire 114.

Expansion of the expandable element 12 may be triggered by any stimulus disclosed herein, in particular ultrasound, electricity, electromagnetic radiation (preferably UV radiation), heat, hydration.

It will be understood that any attachment element described herein, in particular biological, mechanical and chemical attachment mechanisms, may be used in combination with the catheter devices shown in Figs. 19a-19d.

Figs. 20a-20f show different schematically embodiments of attachment elements 4. It will be understood that any of the attachment elements 4 shown here may be combined with any of the catheter devices of microrobot-based devices disclosed herein.

Fig. 20a shows an attachment element 4 comprising an adhesive composition 10.

Fig. 20b shows an attachment element 4 comprising a fork-like mechanical attachment mechanism 11.

Fig. 20c shows an attachment element 4 comprising a thrombogenic material 7.

Fig. 20d shows an attachment element 4 comprising a suction mechanism 115.

Fig. 20e shows an attachment element 4 comprising thrombogenic fibers 7. Thrombogenic fibers 7 may be particularly advantageous the fibers may adapt the shape of the proximal face of the thrombus and provide particularly secure attachment.

Fig. 20f shows an attachment element 4 comprising electrical contacts 8 which may be used to induce electro-thrombosis and/or cauterization.

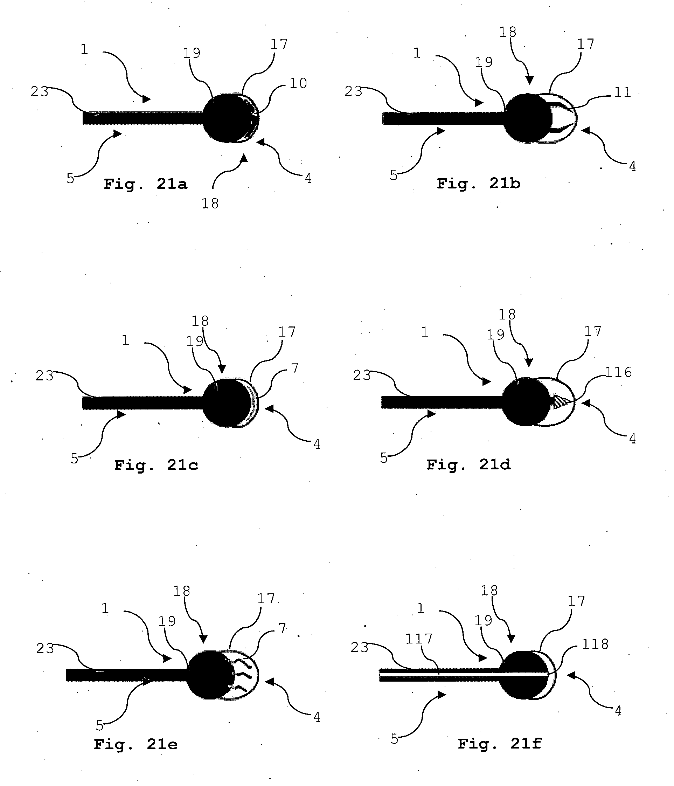

Figs. 21a-21f show schematically different devices 1 according to the invention configured as microrobots 18 comprising a magnetic part 19 attached to a controlling line 23 at a distal end of the device 5. All embodiments shown here comprise a protective layer 17 configured as a biodegradable polymer layer which prevents contact between the attachment element 4 and blood during delivery. It will be understood that while only the delivery configuration is shown here for illustration, degradation of the protective layer 17 release the attachment element 4 and may provide attachment to a thrombus by the ways generally described herein. Alternatively, a non-biodegradable polymer may be used as a protective layer 17.

Fig. 21a shows an embodiment wherein an adhesive composition 10 is arranged on the magnetic part 19 and protected by the protective layer 17.

Fig. 21b shows an embodiment with a fork-like mechanical attachment element 11 attached to the magnetic part 19.

Fig. 21c shows an embodiment with a thrombogenic element 7 attached to the magnetic part 19.

Fig. 21d shows an embodiment with a screw-like element 116 which is particularly suitable to drill and screw into a thrombus and provide attachment. A rotating external magnetic field may be used to rotate the drill-like element 116. For example, a permanent magnet may be rotated to generate such a rotating magnetic field.

Fig. 21e shows an embodiment with fibers 7 that act as a biological attachment element. The fibers 7 may, for example, be PTFE fibers coated with thrombin. Thus, when in contact with blood, coagulation can be triggered, wherein the formed clot can attach to a thrombus to be retrieved. Fibers 7 are particularly advantageous because fibers may adapt to the shape of the proximal face of the thrombus to be retrieved. Additionally or alternatively, the fibers 7 may be coated with a dried adhesive composition. The dried adhesive may be reactivated when in contact with a fluid such as blood. Thus, an adhesive attachment may be provided once the fibers 7. come in contact with the thrombus.

Fig. 21f shows an embodiment wherein a canal 117 is arranged within the controlling line 23. The canal 117 is in fluid communication with an opening 118 arranged at the distal-most end of the magnetic part 19. After removal of the protective layer 17, the opening 118 and canal 117 may be used to provide suction and remove elements from a treatment site. The features of the embodiment shown here is particularly suited to be combined with the embodiment of Fig. 21f. A drill-like element 116 may remove parts of a thrombus that can immediately be removed and transported away by means of a suction action provided by the canal 117 and opening 118.

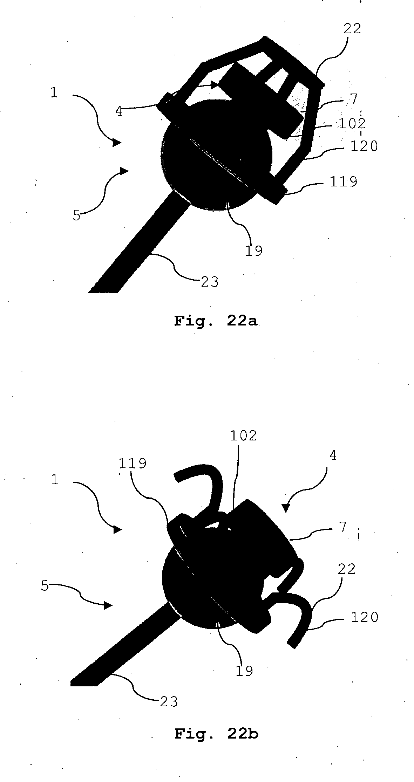

Fig. 22a and 22b show an embodiment of a device 1 comprising a frame 22 as a protective element. For illustration, here, a device 1 comprising a biological attachment element 7 is shown. It will be understood that a frame 22 as shown here may be used in combination with any device 1 disclosed herein. Furthermore, it will be understood that further protective elements, such as protective layers, protective caps, and others, may be used in combination with the frame 22 shown here. In particular, a coating may be used on the frame 22 to prevent blood contact.

Fig. 22a shows the device 1 in a delivery configuration. The frame comprises a ring 119 from which four bars 120 extend in a distal direction and connect at a distance distally of the device 1. Additionally or alternatively, rods may be used. Thus, the frame prevents accidental contact of tissue with the attachment element 4.

Fig. 22b shows the device 1 of Fig. 22a, wherein the frame 22 has been opened. The connection between bars 120 has been removed and the bars 120 extend away from the ring 119. Thus, the attachment element 4 is exposed and can be brought in contact with tissue.

The frame 22 is preferably activatable. The frame 22 is generally composed of a straight frame element fixed to the ring 119 and an activable element which may open the frame upon activation.

Preferably, a polymer film is arranged on the frame 22. Particularly preferably, a cap as shown in Figs. 16 and/or 17 is arranged on the frame 22.

Preferably, the straight elements can comprise or consist of a polymer, a ceramic or a metal. Particularly preferably, stainless steel (316 L), nitinol and/or titanium are used.

The diameter of the bars 120 of the frame 22 may be in the range of 80 µm and 300 µm, preferably 100 µm and 200 µm.

Preferably, the frame 22 is welded, in particular the connections between bars 120 and ring 119.

An external diameter of the frame 22 is preferably in the range of 80 µm to 400 µm, particularly preferably 100 µm to 200 µm. An inner diameter of the hollow frame may be in the range of 50 µm to 300 µm, preferably 70 µm and 150 µm.

Preferably, an electrical current is used to open frame 22. As a result, the bars 120 can bend towards a radial direction of the tool support 102. For example, the bending of bars 120 may tear and open the polymer layer.

The bars 120 of the frame 22 can be bent at an angle in the range of 10° to 170°, preferably 80° to 130°, with respect to a longitudinal axis, of the device 1 (i.e. an axis perpendicular of a plane of ring 119).

In some embodiments, the ring may be arranged at an extremity of a protective element, for example the frame 22 and/or a protective cap as described herein, for reinforcement of the protection. Said ring may break upon bending of the bars 120, for example. Said ring may comprise or consist of bioresorbable polymers such as PGA or PLGA. Additionally or alternatively, the ring comprise or consist of a non-biodegradable polymer or a biodegradable polymer. Said ring may comprise a predetermined breaking point to ensure a smooth breaking. For example, the predetermined breaking point may comprise a local thickness reduction. Said ring may have a diameter in the range of 100 µm and 1000 µm, preferably 150 µm and 250 µm.

It is conceivable that multiple triggers/activation mechanisms are realized in one device. For example, one of several activable elements may be triggered with an electric current whereas other activatable elements may be triggered by an increase in temperature.

Preferably, a protective layer is arranged on the frame 22. The protective frame 22 allows to minimize direct contact of the attachment element with blood vessel walls.

Fig. 23 shows an exemplary embodiment of a device 1 with an additional propulsion mechanism. For illustration and clarity, here, a device 1 without an attachment element is shown. It will be understood that the propulsion features described here may be combined with any device 1 disclosed herein.

The additional propulsion system shown here is chemical. The propulsion system comprises a reactive specie, which is adapted to react with water or blood to form a gas may be used to propel the device. Here, magnesium is used.

The magnesium (not visible) is embedded into a tank 24. The tank 24 here is configured as a tubing setup around the magnetic part 19 and attached to a shell 121. The shell 121 may be a protective coating as described herein, for example a resin shell. The tank 24 is closed with a plug 104. The plug is removably attached to the tank 24 and can be removed mechanically and/or degraded by means of a stimulus. For example, the plug 104 may be attached to a line associated with the controlling line 23. A force exerted on said line, induced for example by a controlling line driver, may remove the plug 104 and thus the content of the tank, here magnesium, can come in contact with blood. As a result, a gas is formed and propelled through opening 103 of the tank, thus providing a forward force on the device 1.

Here, the plug 104 is made of titanium. However, any metal, for example tungsten or steel, may be used, and/or any polymer such as silicon, PEEK, or any blend or combination thereof.

Here, an outer diameter of the plug 104 is 240 µm. An inner diameter of the tank 24 and opening 103 is 100 µm. An outer diameter of the tank is 200 µm.

Preferably, the tank comprises or consists of a polymer material such as PDMS. Other polymers may be used, as well as any blend or combination of polymers.

Figs. 24a and 24b show an alternative embodiment of a device 1 comprising a suction mechanism. Here, the suction mechanism is configured, for illustration, as an attachment mechanism 4. However, it will be understood that such a suction mechanism as shown here may be used in combination with any other attachment mechanism disclosed herein, in particular to provide further attachment in combination with other attachment means and/or to remove debris or thrombus residue to improve the safety of the treatment.

Here, a variant of mechanical attachment is performed by suction. The aspiration system comprises a suction surface 122 arranged on the tool support 102. The tool support 102 further comprises an opening 118 through which a suction action can be achieved. The tool support 102 may in particular have a flat shape, a curved shape, and/or comprise peripheral protrusions to provide sufficient fit with a shape of the thrombus. Here, a diameter of the opening 118 is 600 µm. The thrombus (not shown) can be pulled onto the suction surface 122 and be held by means of the suction on the tool support 102. A suction mechanism as shown here allows to attach and reattach a thrombus repeatedly, for example until secure attachment is provided.

Fig. 24b shows the device of Fig. 24a in a cross-sectional view in a plane parallel to a longitudinal axis of the device. Here, a hollow channel 117 arranged through the controlling line 23 and the magnetic part 19 is visible. The inner diameter of the channel 117 is in the range of 50 µm to 800 µm, preferably 70 µm to 200 µm. When used, the suction mechanism is preferably activated when in proximity to the thrombus.

The hollow channel 117 here is configured as a channel within the controlling line 23, the controlling line 23 serves as a suction channel. The suction channel may, alternatively, be configured as a separate element not incorporated in the controlling line, in particular when combined with a catheter device (see Figs. 19a-19d).

It will also be understood that the hollow line 117 may be used for other purposes than to provide a suction mechanism. In particular, it may be used for hydraulic activation, activation by gas-compression, or delivery of other fluids.

The Young's modulus of the controlling line 23 and/or the channel 117 (in particular if configured as a separate element) may be in the range of 0.5 GPa and 100 GPa, preferably 0.5 GPa and 5 GPa, in order to be compatible with navigation within a vessel system.

In some embodiments configuration, the aspiration system, i.e. the tool support 102, may be directly connected with a hollow tube 117, i.e. without an magnetic part 19 arranged intermediately.

Fig. 25 shows a device 1 configured as a microrobot comprising a magnetic head portion 19 connected to a controlling line 23 and mechanical attachment elements 11. The device 1 is similar to the device described in Fig. 12b. The mechanical attachment elements 11 comprise a Nitinol-based shape memory alloy and are configured to bend outwardly upon heating. In addition, the mechanical attachment elements 11 comprise a shaft 130 each. The shaft 130 is configured to limit the bending of the mechanical attachment element 11. Hence, the shaft 130 may be used to achieve a specific shape for the anchoring, in particular a straight shape in an area close to the tool support 102. Additionally or alternatively, the shaft 130 may exhibit a higher rigidity than the mechanical attachment elements 11. Hence, the shaft may protect the mechanical attachment elements 11 during navigation.

Additionally or alternatively, the shaft 130 may be configured to trigger the movement of the mechanical attachment elements 11. For example, the shaft may be connected to electric wires (see Fig. 9). Hence, the movement of each mechanical attachment element may be controllable by application of a current to the shaft 130.

Here, the shafts 130 are made of stainless steel and have an internal diameter of 200 µm and an outer diameter of 300 µm.

Fig. 26 shows a device 1 which is substantially similar to the devices shown in Figs. 12a-12c. In addition, the mechanical attachment elements comprise a thrombolytic coating 131. Here, the thrombolytic coating 131 is adapted to release tissue plasminogen activator (t-PA). t-PA may soften the thrombus tissue and reduce the required force proximal attachment to a white clot. Furthermore, release of t-PA may reduce the friction force between the thrombus and the blood vessel walls and thus reduce the required force for thrombus retrieval.

It will be understood that the coating 131 might, additionally or alternatively, be made of an expansive material, which can inherently increase the adhesion force between the thrombus and the device.

It will be understood that thrombolytic agents as described here may be used in combination with any other embodiment disclosed herein.

It will be understood that another thrombolytic agents than t-PA may be used in addition or as an alternative.

Thrombolytic agents may be directly grafted on a surface of the medical device, for example chemical vapor deposition (CVD) or physical vapor deposition (PVD).

The thrombolytic agent may also be embedded in a matrix, preferably a polymer matrix. Non-biodegradable polymers such as silicone, PDMS, polyurethane, polyethylene-co-vinyl acetate (PEVA), poly(styrene-b-isobutylene-b-styrene), polybutyl methacrylate (PBMA), poly(vinylidene fluoride-co-hexafluoropropylene), phos-phorylcholine or polyesters may be used. Biodegradable polymers such as PLGA (in any PLA:PGA ratio, preferably 10:90), PDO, PGA, starch, cellulose, and/or chitosan may be used as well.

The matrix can be made of a gel, in particular a hydrogel. The gel may be degradable or non-degradable. The matrix may be arranged on the tool support 102 and held the tool support 102 by the attachment elements, in particular mechanical attachment elements 11.

The thrombolytic matrix may be arranged on a scaffold on the tool support 102. The scaffold may be glued on the tool support. The thrombolytic matrix can exhibit holes for attachment elements. The matrix may have a flat or a curved shape. The shape of the thrombolytic matrix may have an influence on the release of the thrombolytic agent and thus, the release kinetics may be tuned by changing the shape of the thrombolytic matrix.

Typically, a flat surface will lead to comparably slow release of a thrombolytic agent. By contrast, curved surfaces may provide for faster release. For example, a dome shape may be used, in particular with the top oriented toward the thrombus, to achieve this effect.

A shaped scaffold may be used to provide the desired shape. A gel containing the thrombolytic agent can be arranged on it. The curvature may be chosen so that the scaffold encompasses an angle between 10 to 45°, preferably 30°, in particular between a surface of the scaffold and the longitudinal axis of the device.

The thrombolytic matrix is made of different elements distributed on the tool support surface.

A mix of polymers can be used to tune the release kinetics. Low molecular weight polymers can be used to tune the release kinetic.

Typically, polymers with low molecular weight may degrade faster and provide faster release of drugs, thrombogenic agents, thrombolytic agents, or others. Polymers with higher molecular weight may degrade at a slower rate. Thus, the kinetics of release of an active agent may be tuned by mixing polymers with different molecular weights.

Typically, the ratio of thrombolytic agent to polymer (weight by weight) is between 0.1% and 50%, preferably between 2% and 15%.

The thrombolytic matrix may be arranged on any part of the medical device, for example the mechanical attachment elements 11 and/or the tool support 102, and dried.

Additionally or alternatively, the thrombolytic matrix may comprise or consist of particles. For example, the particles may be deposited by electro-spraying. The thrombolytic matrix may also be molded onto the tool support 102. Alternatively, the thrombolytic matrix can be shaped, by molding or casting, into a part to be arranged directly on any part of the device 1, for example on a mechanical attachment element 11, the tool support 102, shaft 130 or others.

The thrombolytic agent can be fixed on any surface of the medical device 1 by, for example, peptide grafting or hydrolysable linkers.

In one method of attaching a thrombolytic agent to a surface, the thrombolytic agent is mixed with a water-soluble polymer such as PVA or PVP. The polymer solution is coated onto a surface and dried.

Any thrombolytic matrix as described herein may be coated onto the mechanical attachment elements 11 in order to create a thrombolytic coating 131.

Fig. 27 shows an alternative embodiment of a medical device 1 which is adapted to release a thrombolytic agent. Here, the thrombolytic agent is embedded in a thrombolytic matrix 132 which is arranged on the tool support 102. The mechanical attachment elements 11 are pierced through the thrombolytic matrix 131.

The thrombolytic matrix 132 may be any thrombolytic matrix as described herein. It will be understood that the mechanical attachment elements 11 shown here, which substantially correspond to the ones shown in Fig. 12a-12c, are of exemplary nature. The thrombolytic matrix 132 shown here may be combined with any medical device 1 described herein.

Any thrombolytic agent may be protected from direct contact with blood during the navigation to a target site. The thrombolytic agent may activated before or at the same time as the attachment element.

Once activated, the thrombolytic agent may be carried by blood up to the thrombus.

Fig. 28 shows an embodiment of a medical device 1 with a mechanical attachment element configured as a spring 11. In addition, fibers 7 are arranged on the spring. The fibers 7 may be adhesive fibers and/or thrombogenic fibers. The fibers 7 provide advantageous attachment as the fibers 7 increase the contact surface with the thrombus (not shown).

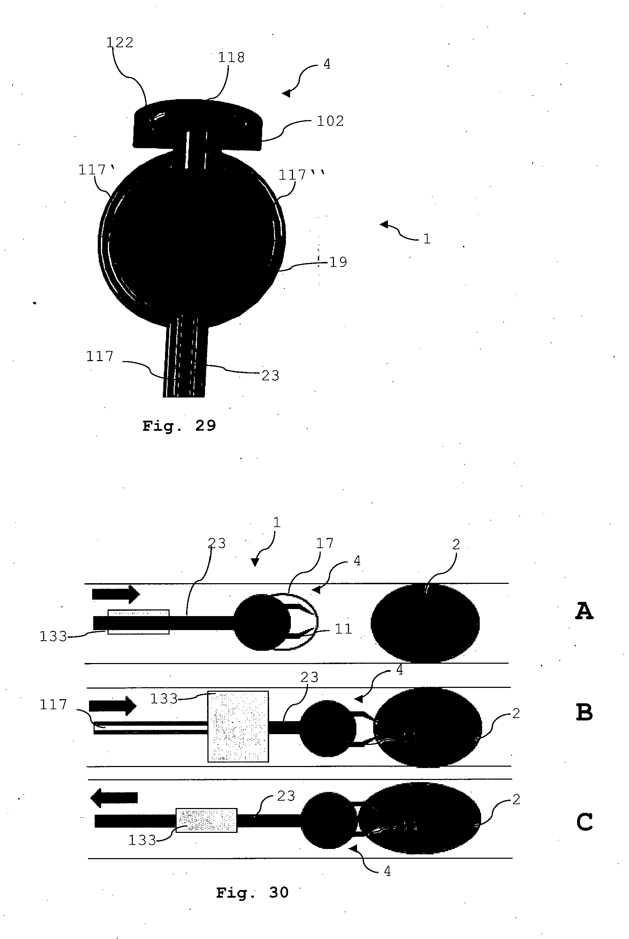

Fig. 29 shows a medical device 1 wherein the attachment element 4 comprises a suction mechanism. The shown embodiment is similar to the embodiment shown in Figs. 24a and 24b. Here, the hollow line 117 in the controlling line 23 does not penetrate the magnetic head part 19. Instead, a Y-fork divides the hollow line 117 into two sub-lines 117',117" which are arranged on the circumference of the magnetic head portion 19. Such an arrangement, the magnetic head portion 19 does not need to be modified. The suction system 117, 118, 122 is connected to at least one hollow line 117.

It is conceivable to use more than one hollow line 117. If several hollow lines are used, at least one line is used for suction. Other lines may be used to activate additional microrobot features, for example for the injection of saline solution. Alternatively, different lines may be connected or connectable to different suction areas. Such a configuration may improve the adhesion to the thrombus.

If one suction orifice 118 is present, a progressive increase of the diameter from the proximal side to the distal side may improve the adhesion to the thrombus.

The inner diameter of hollow sublines 117',117" is preferably 60 µm, and the outer diameter is preferably 90 µm. The diameter of the controlling line shown here is 70 µm. The diameter of the suction orifice 118 increases from 100 µm to 200 µm.

In certain embodiments, the controlling line 23 may be formed by the hollow sublines 117',117''.

Fig. 30 shows a medical device 1 which is substantially similar as the device of Fig. 21b. The device 1 comprises a controlling line 12, a mechanical gripper 11 and protective coating 17. The medical device is shown before activation (A), during attachment (B) and during retrieval (B) of the thrombus (2).

The device 1 shown here further comprises a balloon 133 arranged at a distal position of the attachment element 4 on the controlling line 23. An inside of the balloon 133 is in fluid communication with a hollow line 117 arranged within the controlling line and adapted to inflate the balloon 133. The balloon 133 comprises a silicone pouch.

The balloon 133 allows to reduce the blood flow/blood pressure in an area of the thrombus 2 before and during attachment to mechanical gripper 11. For retrieval of the thrombus 2, the balloon 133 may be deflated again.

The person skilled in the art will understand that all specific combinations of attachment elements, delivery systems, protective coatings and activation mechanisms are of exemplary nature and may be used in any other combination.

In general, conducting polymers may be used instead of Nitinol. Preferred conducting polymers are polythiophene (PT), PA, PPy, PANI, PEDOT.

In general, adhesive compositions as described herein may be used in an encapsulated form.

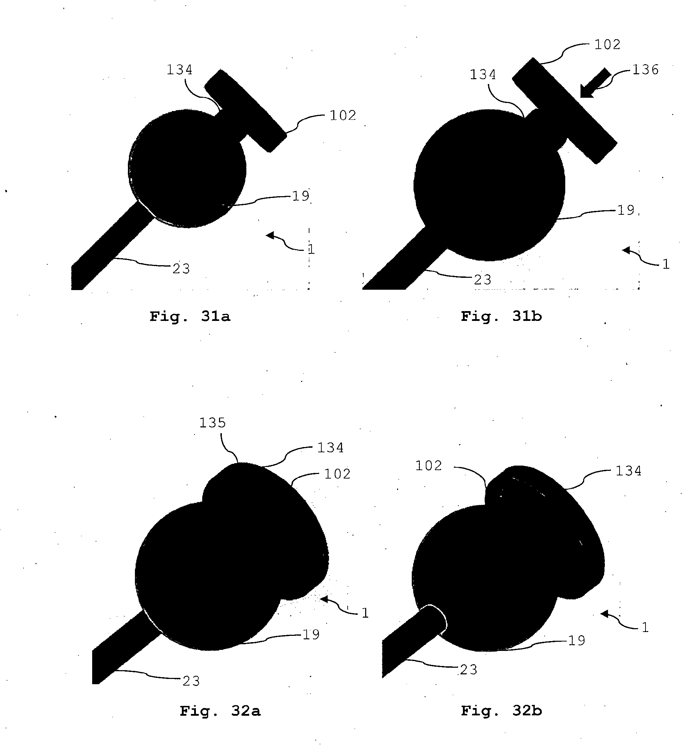

Fig. 31a shows a first embodiment of a device 1 with a sensor 134. The device 1 is substantially similar as other devices described herein, and it will be understood that the sensor 134 may be combined with any device 1 described herein.

Here, the sensor 134 is configured as a pressures sensor. The sensor 134 is arranged between the tool support 102 and the magnetic part 19. Thus, when the tool support 102 is pushed against another structure (for example a thrombus), the force 136 exerted on said structure may be measured by means of the sensor 134. It is in particular conceivable to measure the force via a strain of the sensor.

Fig. 31b shows the embodiment of Fig. 31a in a cross-sectional view.

The sensor 134 may be connected to cables (not shown) and/or comprise a wireless connection to an external device.

Here, the sensor 134 has an external diameter of 300 µm, an internal diameter of 200 µm and a height of 300 µm.

Fig. 32a shows a second embodiment of a device 1 with a sensor 134. Here, the sensor 134 is configured as circumferential ring arranged around the tool support 102. The sensor 134 comprises through-holes 135 through which a fluid, for example blood, can flow. Thus, the sensor 134 is configured as a flow sensor. Preferably, microturbines (not shown) are arranged within the holes 135 to measure the flow velocity of blood relative to the device 1. It is possible to connect the sensor 134 via cables (not shown) or wirelessly.

Fig. 32b shows the device 1 of Fig. 32b from different perspective showing a proximal side of the sensor 134.

## Claims

1. A medical device (1) for retrieving a thrombus (2) from a vessel (3), comprising an attachment element (4), arranged at a distal end (5) of the medical device (1),
wherein the attachment element (4) is adapted to attach to a proximal face (6) of the thrombus (2) such that the thrombus (2) is retrievable by exerting a pulling force (F) on the attachment element (4).

2. The medical device (1) according to claim 1, wherein the attachment element (4) comprises a biological attachment mechanism (7), preferably a thrombogenic element, in particular a thrombogenic element comprising one of thrombin, calcium chloride, and collagen, particularly preferably a dried coagulant layer.

3. The medical device (1) according to any preceding claims,
wherein the attachment element (4) comprises electrical contacts (8) attached or attachable to an electrical conductor (9, 9') and adapted to create attachment through tissue change induced by temperature increase and/or electrical current voltage.

4. The medical device (1) according to any one of the preceding claims, wherein the attachment element (4) comprises a chemical attachment mechanism (10), preferably an adhesive composition, particularly preferably a dried adhesive composition.

5. The medical device (1) according to any one of the preceding claims, wherein the attachment element (4) comprises a mechanical attachment mechanism (11), in particular a mechanical attachment mechanism (11) comprising a shape-change element (12), preferably a mechanical attachment mechanism (11) selected from a group comprising a hook, a fork, a spring, and a spike.

6. The medical device (1) according to any one of the preceding claims, further comprising a suction mechanism (13), preferably wherein the attachment element (4) comprises the suction mechanism.

7. The medical device (1) according to any one of the preceding claims, further comprising an activation mechanism (14;15;16;17), wherein the attachment element (4) has an activated state and a deactivated state, wherein in the deactivated state, the attachment element (4) is adapted to not interact with a vessel wall (3) or with the thrombus (2) and in the activated state the attachment element (4) is adapted to interact with the thrombus (2), wherein the activation mechanism (14;15;16;17;22) is adapted to bring the attachment element (4) at least from the deactivated state to the activated state.

8. The medical device (1) according to any one of the preceding claims, wherein the medical device (1) comprises a microrobot (18) equipped with the attachment element (4).

9. The medical device (1) according to claim 8, wherein the microrobot (18) comprises at least one of a magnetic part (19) and a controlling line (23), wherein the magnetic part (19) is adapted to interact with a, preferably external, magnetic field, and/or wherein the controlling line (23) is attachable or attached to a distal part of the microrobot (18) .

10. The medical device (1) according to any of the preceding claims, wherein the medical device (1) is formed by at least a part of a catheter device (20).

11. The medical device (1) according to claim 7, wherein the activation mechanism (14;15;16;17;22) comprises a mechanism adapted to release at least the attachment element (4) from a storage area (21) of the medical device (1).

12. The medical device (1) according to any one of claims 7 or 11, wherein the activation mechanism (14;15;16;17;22) comprises an activatable material.

13. The medical device (1) according to any one of the preceding claims, wherein the activation mechanism comprises a protection layer (14;15;17;22), preferably a protection layer selected from the group comprising a protection coating (17), a protection sheath (14), a protection frame (22), and a protection cap (15).

14. The medical device (1) according to any one of the preceding claims, further comprising an extension member, preferably a spring (11,12), extendable in an axial direction and adapted for generation of an axial force on the thrombus (2) .

15. The medical device (1) according to any one of the preceding claims, further comprising a sensor (134).

16. The medical device (1) according to any one of the preceding claims, wherein the attachment element (4) comprises a substantially flat surface (F) adapted for attachment to the thrombus (2).

17. The medical device (1) according to any one of the preceding claims, wherein a size (S), preferably a maximum size of the attachment element (4), in a direction (r) perpendicular to a longitudinal axis of the medical device (1) is smaller than 3 mm, preferably smaller than 1 mm, particularly preferably smaller than 0.8 mm, when attached to the thrombus (2).

18. The medical device (1) according to any one of the preceding claims, wherein the attachment element (4) is smaller, in a direction (r) perpendicular to a longitudinal axis (L) of the medical device (1), than a maximum size of the medical device (1), when attached to the thrombus (2).

19. The medical device (1) according to any one of claims 7 to 18, wherein the attachment element (4) is configured such that a maximum size in a direction perpendicular to a longitudinal axis (L) of the medical device differs by less than 10%, preferably less than 5%, particularly preferably remains constant, between the activated and deactivated state.

20. The medical device (1) according to any one of the preceding claims, further comprising a, preferably chemical, propulsion member (24) for generating an axial propulsion force.

21. The medical device (1) according to any one of the preceding claims, further comprising at least one drag member (25), preferably an activatable drag member adapted for increasing forces acting on the medical device.

22. A system comprising a medical device (1), preferably a medical device (1) according to any one of the preceding claims, a controlling unit, and an imaging device, wherein the controlling unit is adapted to navigate the medical device (1) to a target location in a vasculature.

23. A method of producing a medical device (1), preferably a medical device (1) according any one of claims 1 to 20, comprising the steps of
- providing a flexible line with a magnetic part (19) and an attachment element (4) attached thereto;
- coating at least a part of the attachment element (4) with a temporary layer;
- coating at least a part of the medical device (1), preferably with a resin and/or a polymer, particularly preferably including a dipping step and/or a drying step;
- removing the temporary layer.

24. A method of retrieving a thrombus (2) from a vessel with a medical device (1), preferably using a medical device (1) according any one of claims 1 to 20, comprising the steps of
- Introducing a distal end (5) of the medical device (1) in a vessel, the distal end (5) comprising an attachment element (4)
- Navigating the medical device (1) to a target location;
- Optionally, activating an activation mechanism (14;15;16;17;22) such that the attachment element (4) is transformed from a deactivated state to an activated state where it is attachable to the thrombus (2), preferably on a proximal side of the thrombus (2);
- Attaching the attachment element (4) to the thrombus, (2) on a proximal side of the thrombus (2);
- Optionally, stretching the thrombus (2) to reduce the diameter of the thrombus (2) in a cross-section perpendicular to a longitudinal axis of the vessel (3);
- Removing the thrombus (2) from the target site by pulling on said thrombus (2).
